(19) Europäisches Patentamt
European Patent Office
Office européen des brevets

(11) **EP 4 403 555 A1**

(12) **EUROPEAN PATENT APPLICATION**
published in accordance with Art. 153(4) EPC

(43) Date of publication:
**24.07.2024 Bulletin 2024/30**

(21) Application number: **22869371.9**

(22) Date of filing: **16.09.2022**

(51) International Patent Classification (IPC):
***C07D 471/04*** *(2006.01)* ***A61K 31/4375*** *(2006.01)*
***A61P 9/00*** *(2006.01)* ***A61P 9/12*** *(2006.01)*

(52) Cooperative Patent Classification (CPC):
**A61K 31/4375; A61P 9/00; A61P 9/12; C07D 471/04**

(86) International application number:
**PCT/CN2022/119209**

(87) International publication number:
**WO 2023/041004 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.09.2021 CN 202111104458**
**10.11.2021 CN 202111324281**
**29.12.2021 CN 202111636982**

(71) Applicant: **Tuojie Biotech (Shanghai) Co., Ltd.**
**Shanghai 201203 (CN)**

(72) Inventors:
- **LI, Yunfei**
  **Shanghai 201203 (CN)**
- **YU, Jian**
  **Shanghai 201203 (CN)**
- **ZHANG, Zhen**
  **Shanghai 201203 (CN)**
- **LIN, Xiaoyan**
  **Shanghai 201203 (CN)**
- **YANG, Yang**
  **Shanghai 201203 (CN)**
- **DING, Lin**
  **Shanghai 201203 (CN)**

(74) Representative: **Dragotti & Associati S.R.L.**
**Via Nino Bixio, 7**
**20129 Milano (IT)**

# (54) SUBSTITUTED 1,4-DIHYDRO-1,6-NAPHTHYRIDINE AMIDE AND USE THEREOF

(57) The present disclosure relates to a substituted 1,4-dihydro-1,6-naphthyridine amide and a use thereof. Specifically, a compound represented by formula I or a pharmaceutically acceptable salt thereof is provided, where $R^1$, $R^3$-$R^{11}$, $Z^1$ and $Z^2$ are as defined in the present disclosure.

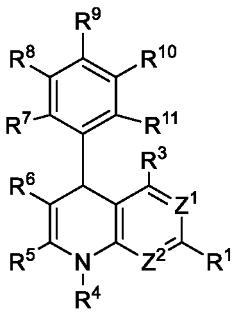

I

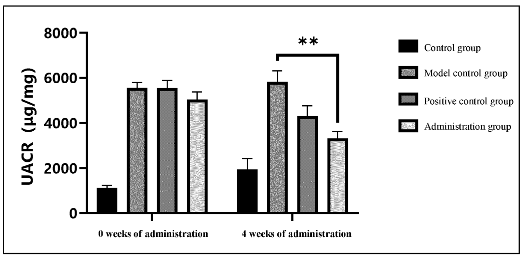


FIG. 1

EP 4 403 555 A1

## Description

### TECHNICAL FIELD

The disclosure pertains to the field of pharmaceutics and relates to a substituted 1,4-dihydro-1,6-naphthyridine amide and use thereof.

### BACKGROUND

Mineralocorticoid receptors (MRs) are aldosterone-activated nuclear hormone receptors that regulate the expression of genes involved in electrolyte homeostasis and cardiovascular diseases. For example, an increase in circulating aldosterone increases blood pressure through its effect on natriuresis and meanwhile potentially affects the brain, the heart, and the vascular system. In addition, hyperaldosteronism is associated with many physiological processes leading to renal and cardiovascular diseases. WO2008104306 reports 4-aryl-1,4-dihydro-1,6-naphthyridine-3-carboxamide derivatives useful as mineralocorticoid receptor antagonists. An exemplary compound is shown below:

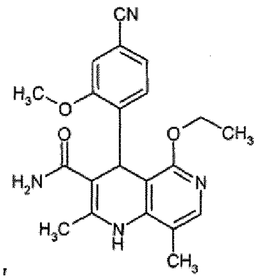
, .

WO2019223629 also reports a class of phenyl-substituted dihydronaphthyridines, which can antagonize mineralocorticoid receptors with an IC50 of up to 3.44 nM. An exemplary compound is shown below:

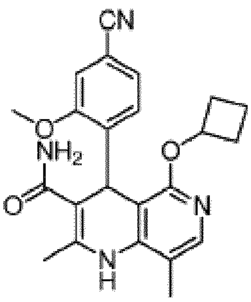
.

There have also been reports about other mineralocorticoid antagonists that have naphthyridine-like structures, e.g., WO2007140934, WO2007140894, and CN202110397352.5.

The compounds of the disclosure are not disclosed in any literature and such compounds exhibit specific MR antagonist effects and good selectivity.

### SUMMARY

The disclosure provides a compound represented by formula I or a pharmaceutically acceptable salt thereof,

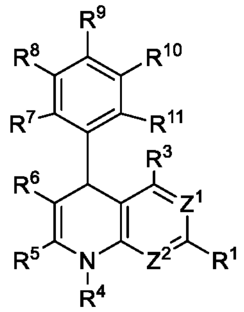

I

wherein $R^1$ is selected from the group consisting of hydrogen, halogen, $C_{1-6}$ alkyl, $C_{1-6}$ alkoxy, 3- to 6-membered cycloalkyl, and 3- to 6-membered heterocycloalkyl; the alkyl, alkoxy, cycloalkyl, or heterocycloalkyl is optionally substituted with one or more $R^{1A}$, and each $R^{1A}$ is independently selected from the group consisting of halogen, hydroxy, cyano, and amino;

$Z^1$ and $Z^2$ are each independently selected from the group consisting of N and C-$R^2$, and $Z^1$ and $Z^2$ are not simultaneously C-$R^2$;

$R^2$ is selected from the group consisting of hydrogen, halogen, $C_{1-6}$ alkyl, $C_{1-6}$ alkoxy, 3-to 6-membered cycloalkyl, and 3- to 6-membered heterocycloalkyl; the alkyl, alkoxy, cycloalkyl, or heterocycloalkyl is optionally substituted with one or more $R^{2A}$, and each $R^{2A}$ is independently selected from the group consisting of halogen, hydroxy, cyano, and amino;

when $Z^1$ is N, $R^3$ is selected from the group consisting of the following functional groups:

1) -S-$C_{1-6}$ alkyl, -S-3- to 6-membered cycloalkyl, and -S-3- to 6-membered heterocycloalkyl, wherein the alkyl, cycloalkyl, or heterocycloalkyl is optionally substituted with halogen, $C_{1-6}$ alkoxy, 3- to 6-membered cycloalkyl, or 3- to 6-heterocycloalkyl;
2) -O-$C_{1-6}$ alkyl, wherein the alkyl is substituted with one or more $R^{8A}$, and each $R^{8A}$ is independently selected from the group consisting of $C_{1-6}$ alkoxy, 3- to 6-membered cycloalkyl, 3- to 6-membered heterocycloalkyl, 3- to 6-membered cycloalkoxy, 3- to 6-membered heterocycloalkoxy, $C_{1-6}$ alkylthio, 3- to 6-membered cycloalkylthio, and 3-to 6-membered heterocycloalkylthio; the alkoxy, cycloalkyl, heterocycloalkyl, cycloalkoxy, heterocycloxy, alkylthio, cycloalkylthio, or heterocycloalkylthio is optionally substituted with halogen, hydroxy, cyano, or amino; and
3) -O-$C_{1-6}$ alkyl, wherein the alkyl is substituted with fluorine at least three times;

when $Z^1$ is C-$R^2$, $R^3$ is selected from the group consisting of halogen, $C_{1-6}$ alkyl, $C_{1-6}$ alkoxy, 3- to 6-membered cycloalkyl, 3- to 6-membered cycloalkoxy, 3- to 6-membered heterocycloalkyl, and 3- to 6-membered heterocycloalkoxy, wherein the alkyl, alkoxy, cycloalkyl, cycloalkoxy, heterocycloalkyl, or heterocycloalkoxy is optionally substituted with one or more $R^{3A}$, and each $R^{3A}$ is independently selected from the group consisting of halogen, hydroxy, cyano, amino, $C_{1-6}$ alkyl, $C_{1-6}$ alkoxy, 3- to 6-membered cycloalkyl, 3- to 6-membered cycloalkoxy, 3- to 6-membered heterocycloalkyl, and 3-to 6-membered heterocycloalkoxy, wherein the alkyl, alkoxy, cycloalkyl, cycloalkoxy, heterocycloalkyl, or heterocycloalkoxy is optionally substituted with one or more groups selected from the group consisting of halogen, hydroxy, cyano, and amino;

$R^4$ is selected from the group consisting of hydrogen, $C_{1-6}$ alkyl, 3- to 6-membered cycloalkyl, and 3- to 6-membered heterocycloalkyl; the alkyl, alkoxy, cycloalkyl, or heterocycloalkyl is optionally substituted with one or more $R^{4A}$, and each $R^{4A}$ is independently selected from the group consisting of halogen, hydroxy, cyano, and amino;

$R^5$ is selected from the group consisting of hydrogen, halogen, $C_{1-6}$ alkyl, $C_{1-6}$ alkoxy, 3-to 6-membered cycloalkyl, and 3- to 6-membered heterocycloalkyl; the alkyl, alkoxy, cycloalkyl, or heterocycloalkyl is optionally substituted with one or more $R^{5A}$, and each $R^{5A}$ is independently selected from the group consisting of halogen, hydroxy, cyano, and amino;

$R^6$ is selected from the group consisting of hydrogen, halogen, $C_{1-6}$ alkyl, NR'(R"), COOR', and CONR'(R"); the alkyl is optionally substituted with one or more $R^{6A}$, and each $R^{6A}$ is independently selected from the group consisting of halogen, hydroxy, cyano, and amino;

R' or R" is independently selected from the group consisting of hydrogen, $C_{1-6}$ alkyl, 3-to 6-membered cycloalkyl, and 3- to 6-membered heterocycloalkyl; the alkyl, alkoxy, cycloalkyl, or heterocyclyl is optionally substituted with one or more groups selected from the group consisting of halogen, hydroxy, cyano, and amino;

$R^7$, $R^8$, $R^9$, $R^{10}$, and $R^{11}$ are independently selected from the group consisting of hydrogen, halogen, hydroxy, cyano, nitro, amino, $C_{1-6}$ alkyl, $C_{1-6}$ alkoxy, 3- to 6-membered cycloalkyl, and 3- to 6-membered heterocycloalkyl; the alkyl, alkoxy, cycloalkyl, or heterocycloalkyl is optionally substituted with one or more $R^{7A}$, and each $R^{7A}$ is

independently selected from the group consisting of halogen, hydroxy, oxo, nitro, cyano, and amino.

In some embodiments, in the compound represented by formula I or the pharmaceutically acceptable salt thereof, $R^1$ is selected from the group consisting of hydrogen, $C_{1-6}$ alkyl, and 3- to 6-membered cycloalkyl; the alkyl or cycloalkyl is optionally substituted with 1-3 $R^{1A}$, and $R^{1A}$ is as defined previously.

In some embodiments, in the compound represented by formula I or the pharmaceutically acceptable salt thereof, $R^1$ is selected from the group consisting of hydrogen and $C_{1-6}$ alkyl, for example, from the group consisting of hydrogen, methyl, difluoromethyl, trifluoromethyl, ethyl, and propyl; further, the alkyl is optionally substituted with 1-3 $R^{1A}$, and $R^{1A}$ is as defined previously.

In some embodiments, in the compound represented by formula I or the pharmaceutically acceptable salt thereof, $R^1$ is selected from the group consisting of halogen, $C_{1-6}$ alkoxy, and 3- to 6-membered heterocycloalkyl; the alkoxy or heterocycloalkyl is optionally substituted with 1-3 $R^{1A}$, and $R^{1A}$ is as defined previously.

In some embodiments, in the compound represented by formula I or the pharmaceutically acceptable salt thereof, $R^1$ is selected from the group consisting of halogen and $C_{1-6}$ alkoxy, for example, from the group consisting of fluorine, chlorine, and methoxy; further, the alkoxy is optionally substituted with 1-3 $R^{1A}$, and $R^{1A}$ is as defined previously.

In some embodiments, when $Z^1$ is C-$R^2$, $R^3$ in the compound represented by formula I or the pharmaceutically acceptable salt thereof is selected from the group consisting of halogen, $C_{1-6}$ alkyl, $C_{1-6}$ alkoxy, and 3- to 6-membered cycloalkyl; the alkyl or cycloalkyl is optionally substituted with 1-3 $R^{3A}$, and $R^{3A}$ is as defined previously.

In some embodiments, when $Z^1$ is C-$R^2$, $R^3$ in the compound represented by formula I or the pharmaceutically acceptable salt thereof is selected from the group consisting of halogen, $C_{1-6}$ alkyl, and $C_{1-6}$ alkoxy; the alkyl or cycloalkyl is optionally substituted with 1-3 $R^{3A}$, and $R^{3A}$ is as defined previously.

In some other embodiments, when $Z^1$ is C-$R^2$, $R^3$ in the compound represented by formula I or the pharmaceutically acceptable salt thereof is selected from the group consisting of $C_{1-6}$ alkyl, 3- to 6-membered cycloalkoxy, 3- to 6-membered heterocycloalkyl, and 3- to 6-membered heterocycloalkoxy; the alkyl, cycloalkoxy, heterocycloalkyl, or heterocycloalkoxy is optionally substituted with 1-3 $R^{3A}$, and $R^{3A}$ is as defined previously.

In some other embodiments, in the compound represented by formula I or the pharmaceutically acceptable salt thereof, $R^3$ is selected from -O-$C_2$ perfluoroalkyl.

In some other embodiments, in the compound represented by formula I or the pharmaceutically acceptable salt thereof, $R^3$ is selected from -O-$C_3$ perfluoroalkyl.

In some other embodiments, in the compound represented by formula I or the pharmaceutically acceptable salt thereof, $R^3$ is selected from -S-$C_{1-6}$ alkyl; the alkyl is optionally substituted with halogen, $C_{1-6}$ alkoxy, 3- to 6-membered cycloalkyl, or 3- to 6-heterocycloalkyl.

In some other embodiments, in the compound represented by formula I or the pharmaceutically acceptable salt thereof, $R^3$ is selected from -S-3- to 6-membered cycloalkyl; the cycloalkyl is optionally substituted with halogen, $C_{1-6}$ alkoxy, 3- to 6-membered cycloalkyl, or 3- to 6- heterocycloalkyl.

In some other embodiments, in the compound represented by formula I or the pharmaceutically acceptable salt thereof, $R^3$ is selected from -O-$C_{1-6}$ alkyl; the alkyl is substituted with one or more $R^{8A}$, and each $R^{8A}$ is independently selected from the group consisting of 3- to 6-membered cycloalkoxy, 3- to 6-membered heterocycloalkoxy, $C_{1-6}$ alkylthio, 3- to 6-membered cycloalkylthio, and 3- to 6-membered heterocycloalkylthio.

In some other embodiments, in the compound represented by formula I or the pharmaceutically acceptable salt thereof, $R^3$ is selected from -O-$C_{1-6}$ alkyl; the alkyl is substituted with one or more $R^{8A}$, and each $R^{8A}$ is independently selected from the group consisting of $C_{1-6}$ alkoxy, 3- to 6-membered cycloalkyl, and 3- to 6-membered heterocycloalkyl.

In some other embodiments, in the compound represented by formula I or the pharmaceutically acceptable salt thereof, $R^3$ is selected from -O-$C_{1-6}$ alkyl; the alkyl is substituted with one or more $R^{8A}$, and each $R^{8A}$ is independently selected from the group consisting of methylthio, difluoromethylthio, trifluoromethylthio, methoxy, difluoromethoxy, and trifluoromethylthio.

In some embodiments, in the compound represented by formula I or the pharmaceutically acceptable salt thereof, $R^6$ is selected from the group consisting of hydrogen, halogen, $C_{1-6}$ alkyl, and NR'(R"), and R' or R" is as defined previously.

In some embodiments, in the compound represented by formula I or the pharmaceutically acceptable salt thereof, $R^6$ is selected from the group consisting of COOR' and CONR'(R"), preferably from CONR'(R"), and R' or R" is as defined previously.

In some embodiments, in the compound represented by formula I or the pharmaceutically acceptable salt thereof, R' or R" is independently selected from the group consisting of hydrogen and $C_{1-6}$ alkyl; the alkyl is optionally substituted with one or more groups selected from the group consisting of halogen, hydroxy, cyano, and amino; further, R' or R" is independently and preferably hydrogen, methyl, difluoromethyl, trifluoromethyl, ethyl, or propyl.

In some embodiments, in the compound represented by formula I or the pharmaceutically acceptable salt thereof, R' or R" is independently selected from the group consisting of hydrogen and 3- to 6-membered cycloalkyl; the

cycloalkyl is optionally substituted with one or more groups selected from the group consisting of halogen, hydroxy, cyano, and amino; further, R' or R" is independently hydrogen, cyclopropyl, or cyclopentyl.

In some other embodiments, in the compound represented by formula I or the pharmaceutically acceptable salt thereof, $R^4$ is selected from the group consisting of hydrogen, $C_{1-6}$ alkyl, and 3- to 6-membered cycloalkyl, preferably from the group consisting of hydrogen, methyl, difluoromethyl, trifluoromethyl, ethyl, propyl, cyclopropyl, and cyclopentyl; further, the alkyl or cycloalkyl is optionally substituted with 1-3 groups selected from the group consisting of halogen, hydroxy, cyano, and amino.

In some embodiments, in the compound represented by formula I or the pharmaceutically acceptable salt thereof, $R^9$ is selected from the group consisting of cyano and nitro.

In some embodiments, in the compound represented by formula I or the pharmaceutically acceptable salt thereof, $R^9$ is selected from the group consisting of hydrogen, halogen, hydroxy, amino, $C_{1-6}$ alkyl, and $C_{1-6}$ alkoxy; the alkyl or alkoxy is optionally substituted with 1-3 $R^{7A}$, and $R^{7A}$ is as defined previously.

In some other embodiments, the compound represented by formula I is:

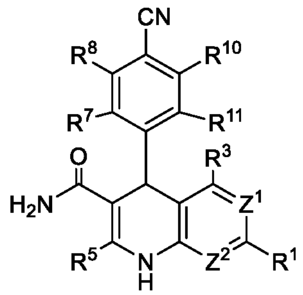

II,

wherein $R^1$, $R^3$, $R^5$, $R^7$, $R^8$, $R^{10}$, $R^{11}$, $Z^1$, and $Z^2$ are as defined in the compound represented by formula I.

In some embodiments, in the compound represented by formula I or formula II or the pharmaceutically acceptable salt thereof, $R^5$ is selected from the group consisting of hydrogen, halogen, $C_{1-6}$ alkyl, and $C_{1-6}$ alkoxy, preferably from the group consisting of hydrogen and $C_{1-6}$ alkyl, for example, from the group consisting of hydrogen, methyl, ethyl, and propyl; further, the alkyl or alkoxy is optionally substituted with 1-3 $R^{5A}$, and $R^{5A}$ is as defined previously.

In some embodiments, in the compound represented by formula I or formula II or the pharmaceutically acceptable salt thereof, $R^7$ is selected from the group consisting of hydrogen, halogen, $C_{1-6}$ alkyl, and $C_{1-6}$ alkoxy, preferably from the group consisting of hydrogen and $C_{1-6}$ alkyl, for example, from the group consisting of hydrogen, $C_{1-6}$ alkyl, and $C_{1-6}$ alkoxy, for example, from the group consisting of hydrogen, methyl, difluoromethyl, trifluoromethyl, ethyl, propyl, methoxy, ethoxy, difluoromethoxy, and trifluoromethoxy; further, the alkyl or alkoxy is optionally substituted with 1-3 $R^{7A}$, and $R^{7A}$ is as defined previously.

In some other embodiments, in the compound represented by formula I or formula II or the pharmaceutically acceptable salt thereof, $R^7$ is selected from the group consisting of hydroxy, amino, 3- to 6-membered cycloalkyl, and 3- to 6-membered heterocycloalkyl. In some embodiments, in the compound represented by formula I or formula II or the pharmaceutically acceptable salt thereof, $R^6$ is selected from the group consisting of hydroxy, amino, cyclopropyl, and cyclopentyl.

In some other embodiments, in the compound represented by formula I or formula II or the pharmaceutically acceptable salt thereof, $R^8$ is selected from the group consisting of hydrogen, halogen, $C_{1-6}$ alkyl, and $C_{1-6}$ alkoxy, preferably from the group consisting of hydrogen and $C_{1-6}$ alkyl, for example, from the group consisting of hydrogen, methyl, difluoromethyl, trifluoromethyl, ethyl, and propyl; further, the alkyl or alkoxy is optionally substituted with 1-3 $R^{7A}$, and $R^{7A}$ is as defined previously.

In some embodiments, in the compound represented by formula I or formula II or the pharmaceutically acceptable salt thereof, $R^8$ is selected from the group consisting of hydroxy, amino, 3- to 6-membered cycloalkyl, and 3- to 6-membered heterocycloalkyl, preferably from the group consisting of hydroxy, amino, cyclopropyl, and cyclopentyl. In another aspect, in the compound represented by formula I or formula II or the pharmaceutically acceptable salt thereof provided by some embodiments, $R^{10}$ is selected from the group consisting of hydrogen, halogen, $C_{1-6}$ alkyl, and $C_{1-6}$ alkoxy, preferably from the group consisting of hydrogen and $C_{1-6}$ alkyl, for example, from the group consisting of hydrogen, methyl, difluoromethyl, trifluoromethyl, ethyl, and propyl; further, the alkyl or alkoxy is optionally substituted with 1-3 $R^{7A}$, and $R^{7A}$ is as defined previously.

In the compound represented by formula I or formula II or the pharmaceutically acceptable salt thereof provided by some embodiments, $R^{10}$ is selected from the group consisting of hydroxy, amino, 3- to 6-membered cycloalkyl, and 3- to 6-membered heterocycloalkyl. In the compound represented by formula I or formula II or the pharmaceutically acceptable salt thereof provided by some other embodiments, $R^{10}$ is selected from the group consisting of hydroxy,

amino, cyclopropyl, and cyclopentyl.

In another aspect, in the compound represented by formula I or formula II or the pharmaceutically acceptable salt thereof provided by some embodiments, $R^{11}$ is selected from the group consisting of hydrogen, halogen, $C_{1-6}$ alkyl, and $C_{1-6}$ alkoxy; the alkyl or alkoxy is optionally substituted with 1-3 $R^{7A}$. In some embodiments, $R^{11}$ is selected from the group consisting of hydrogen, $C_{1-6}$ alkyl, and $C_{1-6}$ alkoxy; the alkyl or alkoxy is optionally substituted with 1-3 $R^{7A}$, for example, hydrogen, methyl, difluoromethyl, trifluoromethyl, ethyl, propyl, methoxy, ethoxy, difluoromethoxy, or trifluoromethoxy. In some other embodiments, in the compound represented by formula I or formula II or the pharmaceutically acceptable salt thereof, $R^{11}$ is selected from the group consisting of hydroxy, amino, 3- to 6-membered cycloalkyl, and 3- to 6-membered heterocycloalkyl, for example, from the group consisting of hydroxy, amino, cyclopropyl, and cyclopentyl.

In another aspect, in the compound represented by formula I or formula II or the pharmaceutically acceptable salt thereof provided by certain embodiments, $Z^1$ is N; $Z^2$ is C-$R^2$, and $R^2$ is as defined previously.

The compound represented by formula I or formula II provided by some embodiments is:

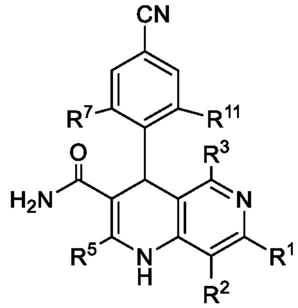
IIIa ,

wherein $R^1$, $R^2$, $R^3$, $R^5$ $R^7$, and $R^{11}$ are as defined in the compound represented by formula I.

In the compound represented by formula I or formula II or the pharmaceutically acceptable salt thereof provided by some other embodiments, $Z^2$ is N; $Z^1$ is C-$R^2$, and $R^2$, $R^2$ is as defined previously.

In some embodiments, the compound represented by formula I or formula II is

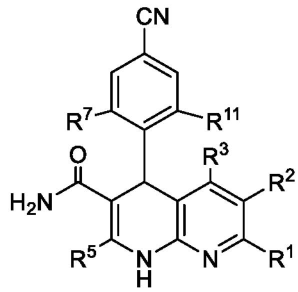
IIIb ,

wherein $R^1$, $R^2$, $R^3$, $R^5$, $R^7$, and $R^{11}$ are as defined in the compound represented by formula I.

In some embodiments, in the compound represented by formula IIIb or the pharmaceutically acceptable salt thereof, $R^3$ is selected from the group consisting of halogen, $C_{1-6}$ alkyl, $C_{1-6}$ alkoxy, and 3- to 6-membered cycloalkyl; the alkyl or cycloalkyl is optionally substituted with 1-3 $R^{3A}$, and $R^{3A}$ is as defined previously.

In some other embodiments, in the compound represented by formula IIIb or the pharmaceutically acceptable salt thereof, $R^3$ is selected from the group consisting of halogen, $C_{1-6}$ alkyl, and $C_{1-6}$ alkoxy; the alkyl or cycloalkyl is optionally substituted with 1-3 $R^{3A}$, and $R^{3A}$ is as defined previously.

In some other embodiments, in the compound represented by formula IIIb or the pharmaceutically acceptable salt thereof, $R^3$ is selected from the group consisting of $C_{1-6}$ alkyl, 3- to 6-membered cycloalkoxy, 3- to 6-membered heterocycloalkyl, and 3- to 6-membered heterocycloalkoxy; the alkyl, cycloalkoxy, heterocycloalkyl, or heterocycloalkoxy is optionally substituted with 1-3 $R^{3A}$, and $R^{3A}$ is as defined previously.

In some embodiments, in the compound represented by formula I or formula II or formula IIIa or formula IIIb or the pharmaceutically acceptable salt thereof, $R^2$ is selected from the group consisting of hydrogen, halogen, $C_{1-6}$ alkyl, and $C_{1-6}$ alkoxy, preferably from the group consisting of hydrogen and $C_{1-6}$ alkyl, for example, from the group consisting of hydrogen, methyl, difluoromethyl, trifluoromethyl, ethyl, and propyl; further, the alkyl or alkoxy is optionally substituted with 1-3 $R^{2A}$, and $R^{2A}$ is as defined previously.

In some embodiments, in the compound represented by formula I or formula II or formula IIIa or formula IIIb or the pharmaceutically acceptable salt thereof, $R^2$ is selected from the group consisting of 3- to 6-membered cycloalkyl and 3- to 6-membered heterocycloalkyl; the cycloalkyl or heterocycloalkyl is optionally substituted with 1-3 $R^{2A}$, and $R^{2A}$ is as defined previously.

In another aspect, in the compound represented by formula I or formula II or formula IIIa or formula IIIb or the pharmaceutically acceptable salt thereof provided by some embodiments, $R^2$ is selected from the group consisting of hydrogen, fluorine, chlorine, difluoromethyl, and trifluoromethyl.

In another aspect, in the compound represented by formula I or formula II or the pharmaceutically acceptable salt thereof provided by some embodiments, $Z^1$ is N; $Z^2$ is N.

In some embodiments, the compound represented by formula I or formula II is

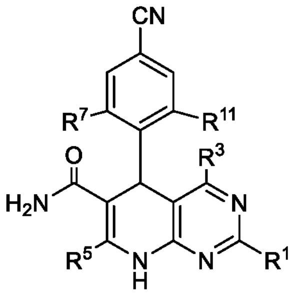

IIIc,

wherein $R^1$, $R^3$, $R^5$, $R^7$, and $R^{11}$ are as defined in the compound represented by formula I.

Further, in some embodiments, in the compound represented by formula I or formula II or the pharmaceutically acceptable salt thereof, each $R^{3A}$ is independently selected from the group consisting of halogen, hydroxy, and $C_{1-6}$ alkyl, preferably from the group consisting of fluorine, chlorine, hydroxy, methyl, ethyl, and propyl.

In some other embodiments, in the compound represented by formula I or formula II or the pharmaceutically acceptable salt thereof, each $R^{3A}$ is independently selected from the group consisting of 3- to 6-membered cycloalkyl, 3- to 6-membered cycloalkoxy, 3-to 6-membered heterocycloalkyl, and 3- to 6-membered heterocycloalkoxy; the cycloalkyl, cycloalkoxy, heterocycloalkyl, or heterocycloalkoxy is optionally substituted with one or more groups selected from the group consisting of halogen and hydroxy.

In some embodiments, in the compound represented by formula I or formula II or the pharmaceutically acceptable salt thereof, $R^{1A}$, $R^{2A}$, $R^{4A}$, and $R^{5A}$ are each independently selected from the group consisting of halogen and hydroxy, preferably from the group consisting of fluorine, chlorine, and hydroxy.

In another aspect, in the compound represented by formula I or formula II or formula IIIa or formula IIIb or formula IIIc or the pharmaceutically acceptable salt thereof provided by some embodiments, $R^1$ is selected from the group consisting of hydrogen, fluorine, chlorine, difluoromethyl, and trifluoromethyl.

Typical compounds provided by the disclosure include, but are not limited to:

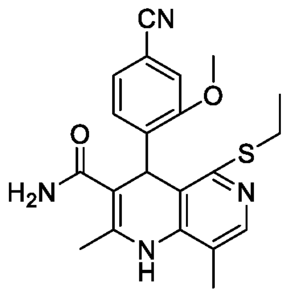

,

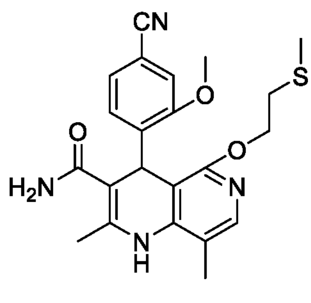

,

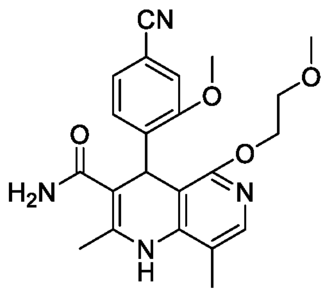

,

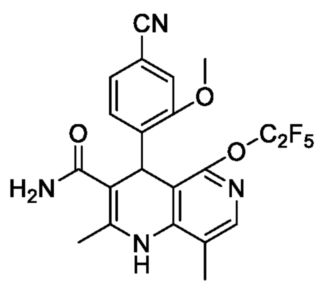 , 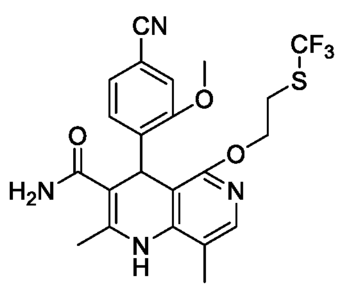 or 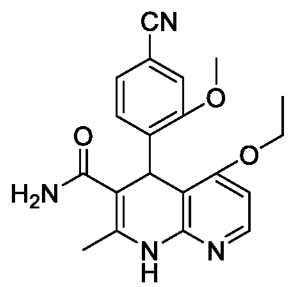 .

In some embodiments, the compound represented by formula I is:

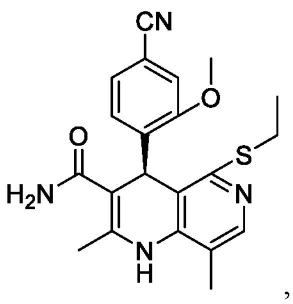 , 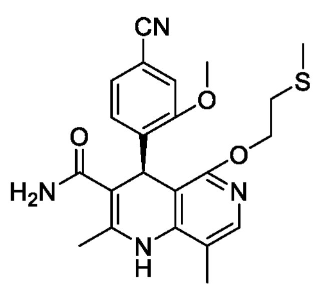 , 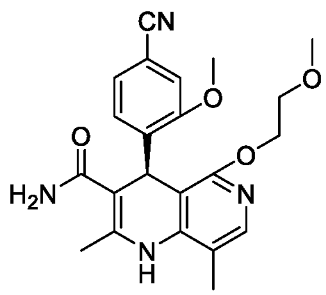 ,

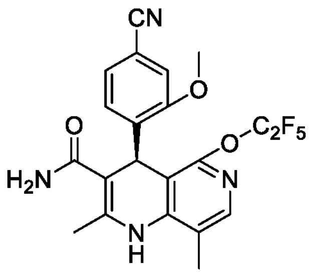 , 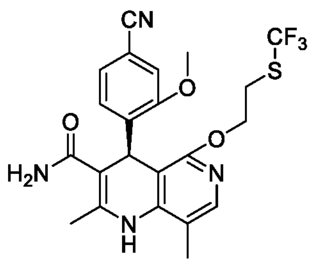 , 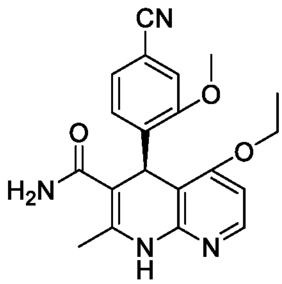 ,

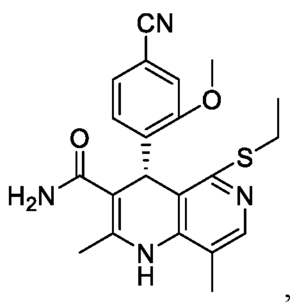 , 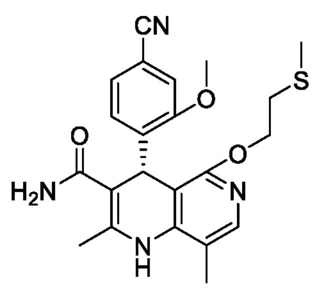 , 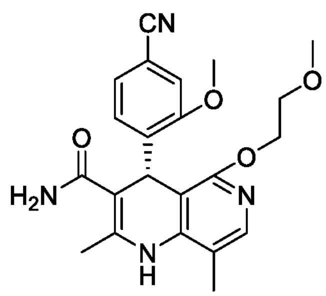 ,

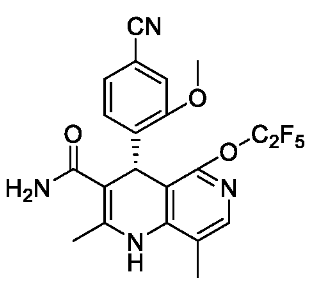 , 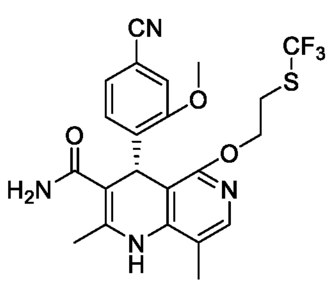 or 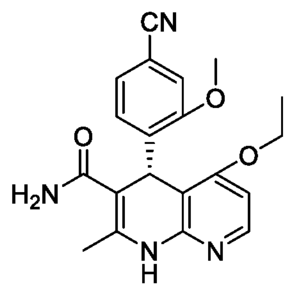 .

In another aspect, the disclosure further provides the following compound or a pharmaceutically acceptable

salt thereof:

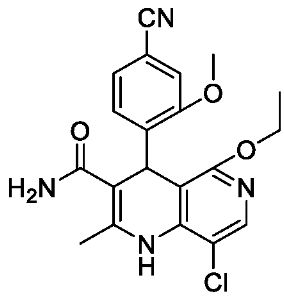,
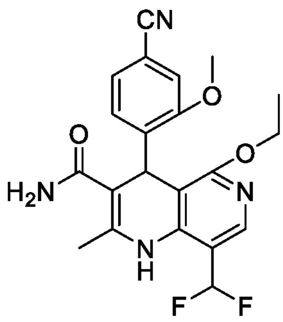,
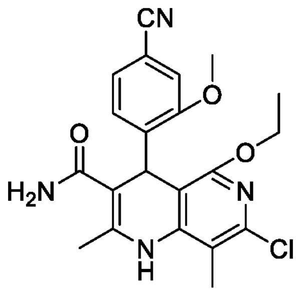,

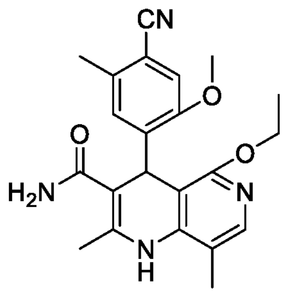,
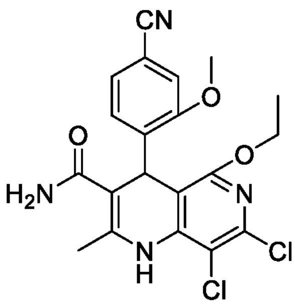,
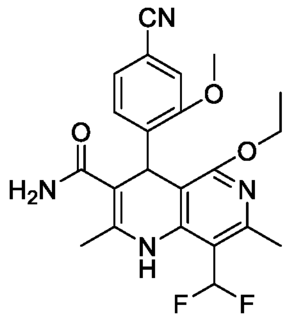,

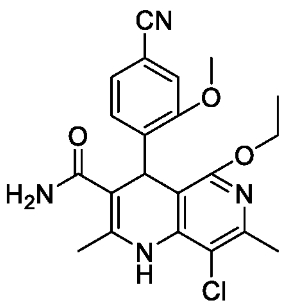,
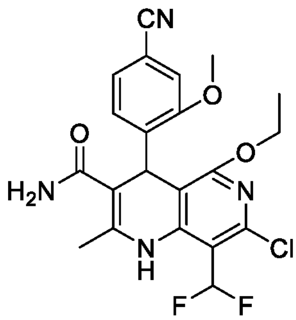,
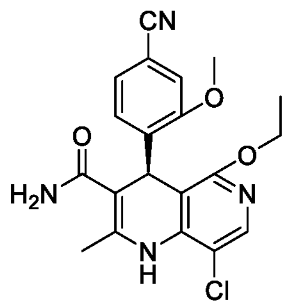,

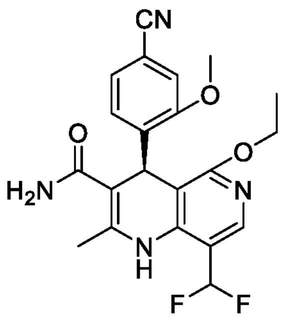,
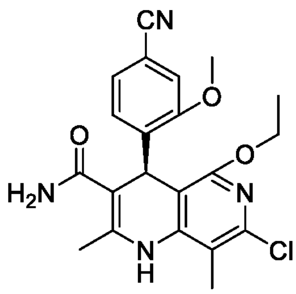,
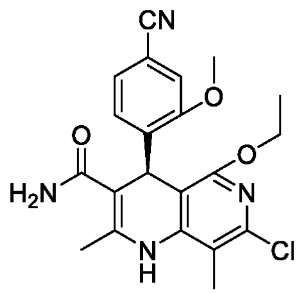,

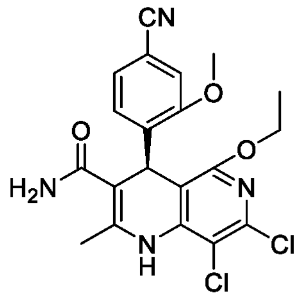
,
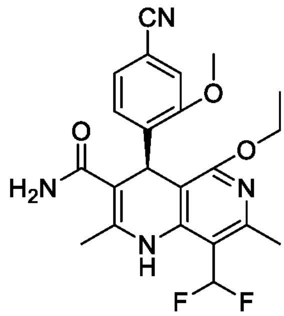
,
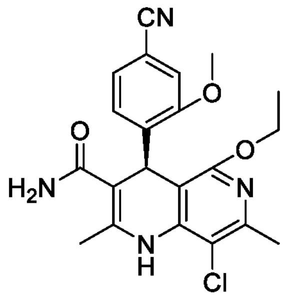
,

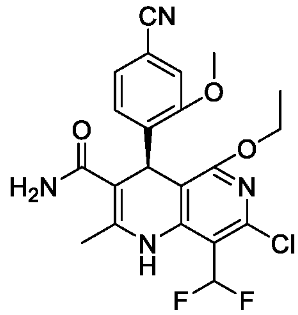
,
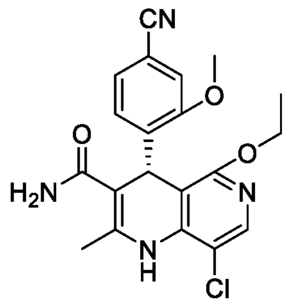
,
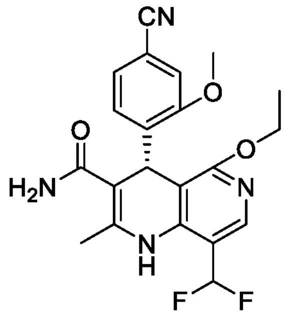
,

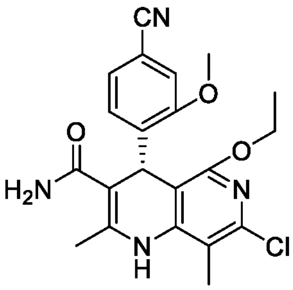
,
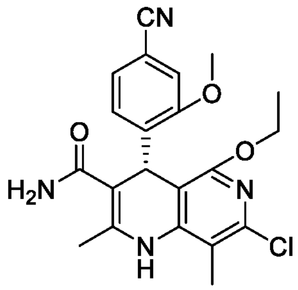
,
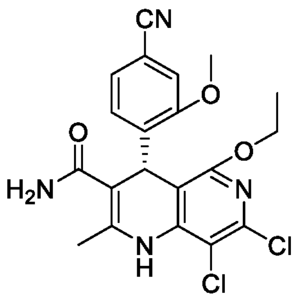
,

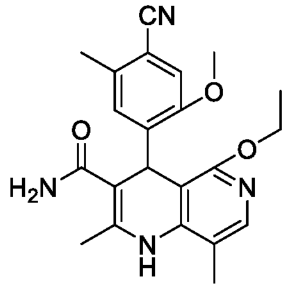
,
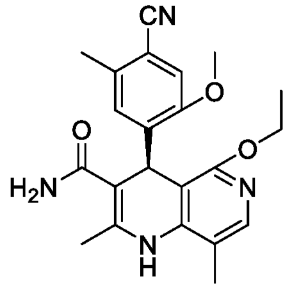
,
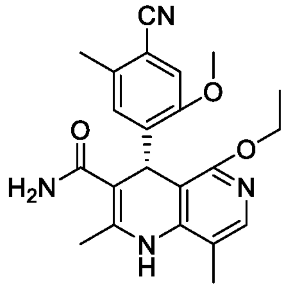
,

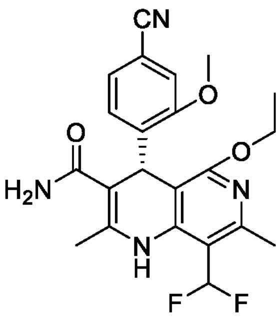
.
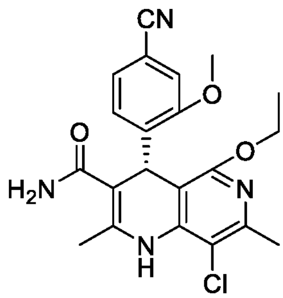
,
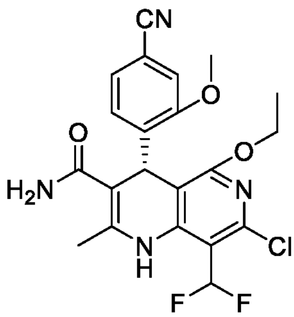
.

, ,

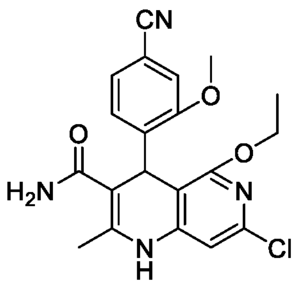,
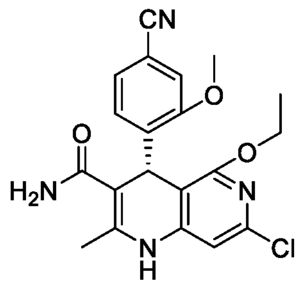 or
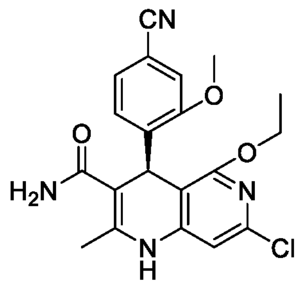.

The disclosure further provides an isotopically substituted form of the aforementioned compound or pharmaceutically acceptable salt thereof. In some embodiments, the isotopically substituted form is a deuterated form.

In another aspect, the disclosure further provides the following compound or a pharmaceutically acceptable salt thereof:

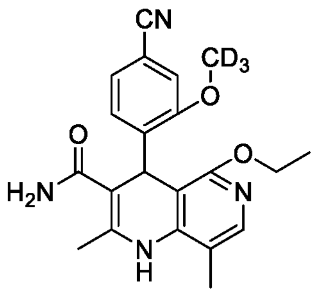,
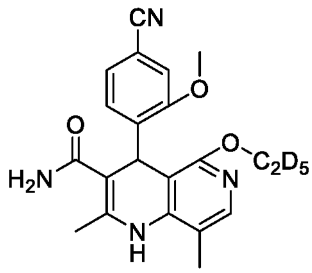,
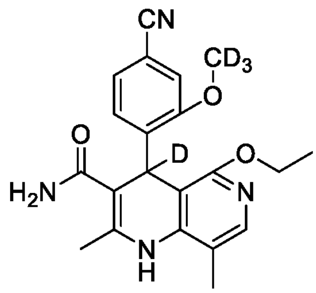,

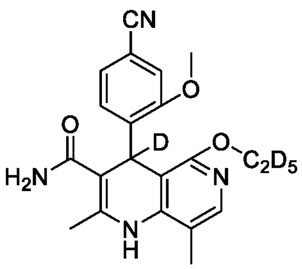,
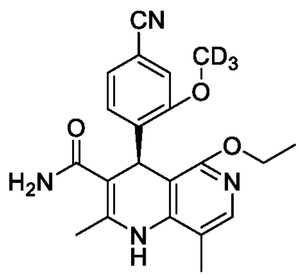,
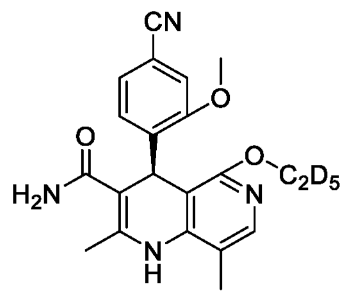,

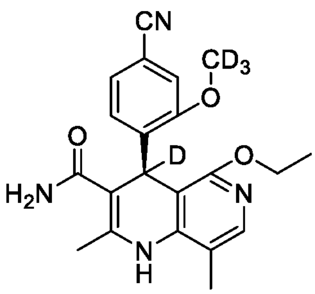,
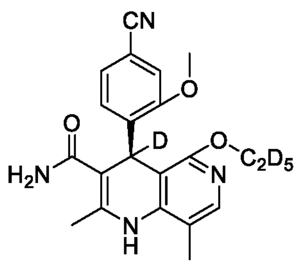,
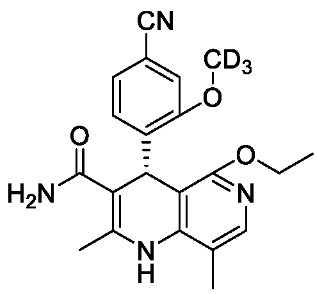,

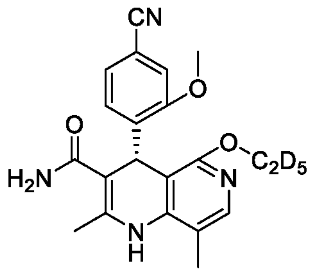, 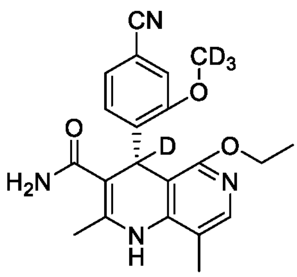 or 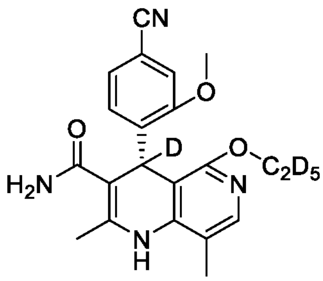.

In *in-vitro* activity assays of compounds in the disclosure, the characteristic of luciferase binding to the substrate to cause a chemiluminescence reaction can be utilized to transfect human embryonic kidney cells (HEK293T) with a plasmid comprising the mineralocorticoid receptor ligand binding domain (LBD) and fused with the Gal4 DNA-binding domain (DBD) and a firefly luciferase reporter gene plasmid under the control of Gal4 UAS (upstream activation sequence). The influence of the presence of stimulation or different stimuli on mineralocorticoid receptor activity is judged by the level of firefly luciferase activity.

The compounds of the disclosure have excellent antagonistic effects on mineralocorticoid receptors (MRs). In some embodiments, the compounds of the disclosure antagonize mineralocorticoid receptors (MRs) with an $IC_{50}$ value of 0.01 to 500 nM. In some embodiments, the compounds of the disclosure antagonize mineralocorticoid receptors (MRs) with an $IC_{50}$ value of 0.01 to 100 nM. In some embodiments, the compounds of the disclosure antagonize mineralocorticoid receptors (MRs) with an $IC_{50}$ value of 0.01 to 20 nM. In some embodiments, the compounds of the disclosure antagonize mineralocorticoid receptors (MRs) with an $IC_{50}$ value of 0.1 to 20 nM. In some embodiments, the compounds of the disclosure antagonize mineralocorticoid receptors (MRs) with an $IC_{50}$ value of 0.1 to 30 nM. In some embodiments, the compounds of the disclosure antagonize mineralocorticoid receptors (MRs) with an $IC_{50}$ value of <50 nM.

The disclosure further provides a pharmaceutical composition, comprising a therapeutically effective amount of at least one of the aforementioned compounds or pharmaceutically acceptable salts or isotopically substituted forms thereof and a pharmaceutically acceptable excipient.

In some embodiments, a unit dose of the pharmaceutical composition is 0.001 mg-1000 mg.

In certain embodiments, the pharmaceutical composition comprises 0.01-99.99% of an aforementioned compound or pharmaceutically acceptable salt or isotopically substituted form thereof based on the total weight of the composition. In certain embodiments, the pharmaceutical composition comprises 0.1-99.9% of an aforementioned compound or pharmaceutically acceptable salt or isotopically substituted form thereof. In certain embodiments, the pharmaceutical composition comprises 0.5%-99.5% of an aforementioned compound or pharmaceutically acceptable salt or isotopically substituted form thereof. In certain embodiments, the pharmaceutical composition comprises 1%-99% of an aforementioned compound or pharmaceutically acceptable salt or isotopically substituted form thereof. In certain embodiments, the pharmaceutical composition comprises 2%-98% of an aforementioned compound or pharmaceutically acceptable salt or isotopically substituted form thereof.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of a pharmaceutically acceptable excipient. In certain embodiments, the pharmaceutical composition comprises 0.5%-99.5% of a pharmaceutically acceptable excipient. In certain embodiments, the pharmaceutical composition comprises 1%-99% of a pharmaceutically acceptable excipient. In certain embodiments, the pharmaceutical composition comprises 2%-98% of a pharmaceutically acceptable excipient.

The disclosure further provides a method for preventing and/or treating a mineralocorticoid-related disease or disorder by administering to the patient a therapeutically effective amount of an aforementioned compound or pharmaceutically acceptable salt or isotopically substituted form thereof or the aforementioned pharmaceutical composition.

In some embodiments, the mineralocorticoid-related disease or disorder is selected from the group consisting of hyperaldosteronism, hypertension, and heart failure.

The disclosure further provides a method for preventing and/or treating a patient afflicted with hyperaldosteronism, hypertension, or heart failure, comprising administering to the patient a therapeutically effective amount of the aforementioned compound represented by formula I or formula II or formula III or pharmaceutically acceptable salt or isotopically substituted form thereof or the aforementioned pharmaceutical composition.

The disclosure further provides use of the aforementioned compounds or pharmaceutically acceptable salts thereof or the aforementioned pharmaceutical composition in the preparation of a medicament for preventing and/or treating a mineralocorticoid-related disease or disorder. In some embodiments, the mineralocorticoid-related disease or disorder is preferably hyperaldosteronism, hypertension, or heart failure.

The disclosure further provides use of the aforementioned compounds or pharmaceutically acceptable salts thereof or the aforementioned pharmaceutical composition in the preparation of a medicament for preventing and/or treating hyperaldosteronism, hypertension, and heart failure.

The pharmaceutically acceptable salts of the compounds described in the disclosure may be selected from the group consisting of inorganic salts and organic salts.

The compounds of the disclosure may exist in specific geometric or stereoisomeric forms. The disclosure contemplates all such compounds, including cis and trans isomers, (-)- and (+)-enantiomers, (R)- and (*S*)-enantiomers, diastereomers, (D)-isomer, (L)-isomer, and racemic mixtures and other mixtures thereof, such as enantiomerically or diastereomerically enriched mixtures, all of which are within the scope of the disclosure. Additional asymmetric carbon atoms may be present in substituents such as an alkyl group. All such isomers and mixtures thereof are included within the scope of the disclosure. The compounds of the disclosure containing asymmetric carbon atoms can be isolated in optically active pure form or in racemic form. The optically active pure form can be isolated from a racemic mixture or synthesized using chiral starting materials or chiral reagents.

Optically active (R)- and (*S*)-enantiomers, and *D*- and *L*-isomers can be prepared by chiral synthesis, chiral reagents or other conventional techniques. If one enantiomer of a certain compound of the disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines). In the chemical structures of the compounds of the disclosure, a bond "" represents an unspecified configuration; that is, if chiral isomers exist in the chemical structures, the bond "" may be "" or "", or contains both the configurations of "" and "".

In the chemical structures of the compounds of the disclosure, a bond "" is not specified with a configuration; that is, they may be in a Z configuration or an E configuration, or contain both configurations.

The compounds and intermediates of the disclosure may also exist in different tautomeric forms, and all such forms are included within the scope of the disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

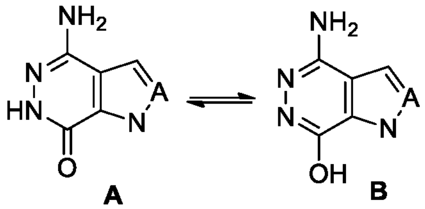

A B

All compounds in the disclosure can be drawn as form A or form B. All tautomeric forms are within the scope of the disclosure. The names of the compounds do not exclude any tautomers.

The disclosure further includes isotopically labeled compounds that are identical to those recited herein but have one or more atoms replaced by an atom having an atomic mass or mass number different from the atomic mass or mass number usually found in nature. Examples of isotopes that can be incorporated into the compounds of the disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The disclosure further includes various deuterated forms of the compound represented by formula (I). Each available hydrogen atom connected to a carbon atom may be independently replaced by a deuterium atom. Those skilled in the art are able to synthesize the deuterated forms of the compound of formula (I) according to the relevant literature.

Commercially available deuterated starting materials can be used in preparing the deuterated forms of the

compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. "Optionally" or "optional" means that the event or circumstance subsequently described may, but does not necessarily, occur and that the description includes instances where the event or circumstance occurs or does not occur. For example, "$C_{1-6}$ alkyl that is optionally substituted with a halogen or cyano" means that the halogen or cyano may, but does not necessarily, exist, and the description includes the instance where alkyl is substituted with a halogen or cyano and the instance where alkyl is not substituted with a halogen or cyano.

Terms and definitions:

"Pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activity.

"Pharmaceutically acceptable excipient" includes, but is not limited to, any adjuvant, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

"Effective amount" or "therapeutically effective amount" described in the disclosure includes an amount sufficient to ameliorate or prevent a symptom or disorder of a medical disorder. An effective amount also refers to an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

"Alkyl" refers to a saturated aliphatic hydrocarbon group, including linear and branched groups of 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof, and the like. Alkyl may be substituted or unsubstituted, and when it is substituted, the substituent may be substituted at any accessible point of attachment, preferably one or more of the following groups, independently selected from the group consisting of halogen, hydroxy, cyano, amino, $C_{1-6}$ alkyl, $C_{1-6}$ alkoxy, 3- to 6-membered cycloalkyl, 3- to 6-membered cycloalkoxy, 3- to 6-membered heterocycloalkyl, and 3- to 6-membered heterocycloalkoxy; the alkyl, alkoxy, cycloalkyl, cycloalkoxy, heterocycloalkyl, or heterocycloalkoxy is optionally substituted with one or more groups selected from the group consisting of halogen, hydroxy, cyano, and amino.

The term "cycloalkyl" refers to a saturated or partially unsaturated, monocyclic or polycyclic hydrocarbon substituent; the cycloalkyl ring contains 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, and the like. Polycyclic cycloalkyl includes spirocycloalkyl, fused cycloalkyl, and bridged cycloalkyl. Cycloalkyl may be substituted or unsubstituted, and when it is substituted, the substituent may be substituted at any accessible point of attachment, preferably one or more of the following groups, independently selected from the group consisting of halogen, hydroxy, cyano, amino, $C_{1-6}$ alkyl, $C_{1-6}$ alkoxy, 3- to 6-membered cycloalkyl, 3- to 6-membered cycloalkoxy, 3- to 6-membered heterocycloalkyl, and 3- to 6-membered heterocycloalkoxy; the alkyl, alkoxy, cycloalkyl, cycloalkoxy, heterocycloalkyl, or heterocycloalkoxy is optionally substituted with one or more groups selected from the group consisting of halogen, hydroxy, cyano, and amino.

The term "heterocycloalkyl" refers to a saturated or partially unsaturated monocyclic or polycyclic hydrocarbon substituent containing 3 to 6 ring atoms. Non-limiting examples of "heterocycloalkyl" include:

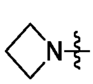 , 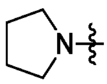 , 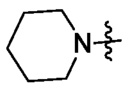 ,

or the like. Heterocycloalkyl may be optionally substituted or unsubstituted, and when it is substituted, the substituent may be one or more of the following groups independently selected from the group consisting of halogen, hydroxy, cyano, amino, $C_{1-6}$ alkyl, $C_{1-6}$ alkoxy, 3- to 6-membered cycloalkyl, 3- to 6-membered cycloalkoxy, 3- to 6-membered heterocycloalkyl, and 3- to 6-membered heterocycloalkoxy; the alkyl, alkoxy, cycloalkyl, cycloalkoxy, heterocycloalkyl, or heterocycloalkoxy is optionally substituted with one or more groups selected from the group consisting of halogen, hydroxy, cyano, and amino.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted, and when it is substituted, the substituent may be one or more of the following groups independently selected from the group consisting of halogen, hydroxy, cyano, amino, $C_{1-6}$ alkyl, $C_{1-6}$ alkoxy, 3- to 6-membered cycloalkyl, 3- to 6-membered cycloalkoxy, 3- to 6-membered heterocycloalkyl, and 3- to 6-membered heterocycloalkoxy; the alkyl, alkoxy, cycloalkyl, cycloalkoxy, heterocycloalkyl, or heterocycloalkoxy is optionally substituted with one or more groups selected from the group consisting of halogen, hydroxy, cyano, and amino.

Likewise, the definitions of "cycloalkoxy" and "heterocycloalkoxy" are similar to the above definition of "alkoxy".

The term "alkylthio" refers to -S-(alkyl), wherein the alkyl is as defined above. Non-limiting examples of alkoxy include: methylthio, ethylthio, propylthio, and butylthio. Alkylthio may be optionally substituted or unsubstituted, and when it is substituted, the substituent is preferably one or more of the following groups independently selected from the group consisting of $C_{1-6}$ alkoxy, 3- to 6-membered cycloalkyl, 3- to 6-membered heterocycloalkyl, 3- to 6-membered cycloalkoxy, 3- to 6-membered heterocycloalkoxy, $C_{1-6}$ alkylthio, 3- to 6-membered cycloalkylthio, and 3-to 6-membered heterocycloalkylthio; the alkoxy, cycloalkyl, heterocycloalkyl, cycloalkoxy, heterocycloxy, alkylthio, cycloalkylthio, or heterocycloalkylthio is optionally substituted with halogen, hydroxy, cyano, or amino.

Likewise, the definitions of "cycloalkylthio" and "heterocycloalkylthio" are similar to the above definition of "alkylthio". The term "hydroxy" refers to the -OH group.

The term "halogen" refers to fluorine, chlorine, bromine or iodine.

The term "cyano" refers to -CN.

The term "amino" refers to $-NH_2$.

The term "nitro" refers to $-NO_2$.

The term "oxo" refers to the =O substituent.

"Substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort.

## BRIEF DESCRIPTION OF THE DRAWING

FIG. 1 shows the urine albumin-to-creatinine ratios (UACRs) of groups of mice.

## DETAILED DESCRIPTION

The disclosure is further described below using examples. However, these examples do not limit the scope of the disclosure.

Experimental procedures without conditions specified in the examples of the disclosure were generally conducted according to conventional conditions, or according to conditions recommended by the manufacturers of the starting materials or commercial products. Reagents without specific origins indicated are commercially available conventional reagents.

The structures of compounds were determined by nuclear magnetic resonance (NMR) spectroscopy and/or mass spectrometry (MS). The NMR shifts ($\delta$) are given in $10^{-6}$ (ppm). The NMR analyses were performed on a Bruker AVANCE-400 nuclear magnetic resonance instrument, with dimethyl sulfoxide-D6 (DMSO-$d_6$), chloroform-D ($CDCl_3$), and methanol-D4 ($CD_3OD$) as solvents and tetramethylsilane (TMS) as an internal standard. The spatial configurations of the optical isomers (isomers) of the compounds can be further confirmed by measuring single crystal parameters.

The HPLC analyses were performed on Waters ACQUITY ultra high performance LC, Shimadzu LC-20A systems, Shimadzu LC-2010HT series, or Agilent 1200 LC high performance liquid chromatograph (ACQUITY UPLC BEH C18 1.7 $\mu$m 2.1 $\times$ 50 mm column, Ultimate XB-C18 3.0 $\times$ 150 mm column, or Xtimate C18 2.1 $\times$ 30 mm column).

The MS analyses were performed on a Waters SQD2 mass spectrometer in the positive/negative ion scan mode with a mass scan range of 100-1200.

The Chiral HPLC analyses were performed using a Chiralpak IC-3 100 $\times$ 4.6 mm I.D., 3 $\mu$m; Chiralpak AD-3 150 $\times$ 4.6 mm I.D., 3 $\mu$m; Chiralpak AD-3 50 $\times$ 4.6 mm I.D., 3 $\mu$m; Chiralpak AS-3 150 $\times$ 4.6 mm I.D., 3 $\mu$m; Chiralpak AS-3 100 $\times$ 4.6 mm I.D., 3 $\mu$m; ChiralCel OD-3 150 $\times$ 4.6 mm I.D., 3 $\mu$m; Chiralcel OD-3 100 $\times$ 4.6 mm I.D., 3 $\mu$m; ChiralCel OJ-H 150 $\times$ 4.6 mm I.D., 5 $\mu$m; or Chiralcel OJ-3 150 $\times$ 4.6 mm I.D., 3 $\mu$m column.

Yantai Huanghai HSGF254 or Qingdao GF254 silica gel plates, 0.15 mm-0.2 mm layer thickness, were used in the thin-layer chromatography (TLC) analyses and 0.4 mm-0.5 mm layer thickness in the thin-layer chromatography separations and purifications.

The flash column purifications were performed using Combiflash Rf150 (TELEDYNE ISCO) or Isolara one (Biotage).

In the normal-phase column chromatography purifications, a 100-200 mesh, 200-300 mesh, or 300-400 mesh Yantai Huanghai silica gel was generally used as a carrier, or a Changzhou Santai pre-fill ultrapure normal-phase silica gel column (40-63 μm, 60 g, 12 g, 25 g, 40 g, 80 g, or other specifications) was used.

In the reversed-phase column chromatography purifications, a Changzhou Santai pre-fill ultrapure C18 silica gel column (20-45 μm, 100 Å, 40 g, 80 g, 120 g, 220 g, or other specifications) was generally used.

The high-pressure column purifications were performed using Waters AutoP equipped with a Waters XBridge BEH C18 OBD Prep Column, 130 Å, 5 μm, 19 mm × 150 mm or Atlantis T3 OBD Prep Column, 100 Å, 5 μm, 19 mm × 150 mm.

In the preparative chiral chromatography purifications, a DAICEL CHIRALPAK IC (250 mm × 30 mm, 10 μm) or Phenomenex-Amylose-1 (250 mm × 30 mm, 5 μm) column was used.

Known starting materials in the disclosure may be synthesized using or according to methods known in the art, or may be purchased from Shanghai Titan Scientific, ABCR GmbH & Co. KG, Acros Organics, Aldrich Chemical Company, Accela ChemBio Inc., Darui Chemicals, and other companies.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization/hydrogen filling.

The microwave reactions were performed using a CEM Discover-S 908860 microwave reactor.

In the examples, the solutions refer to aqueous solutions unless otherwise specified.

In the examples, the reaction temperature is room temperature, i.e., 20 °C-30 °C, unless otherwise specified.

The monitoring of the reaction processes in the examples was conducted using thin-layer chromatography (TLC). The developing solvents for the reactions, the eluent systems for the column chromatography purification of the compounds, the developing solvent systems for thin-layer chromatography, and the volume ratio of the solvents were adjusted depending on the polarity of the compound, or adjusted by adding a small amount of basic or acidic reagents such as triethylamine and acetic acid.

Example 1

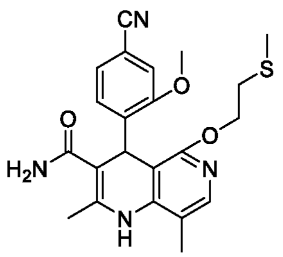

1

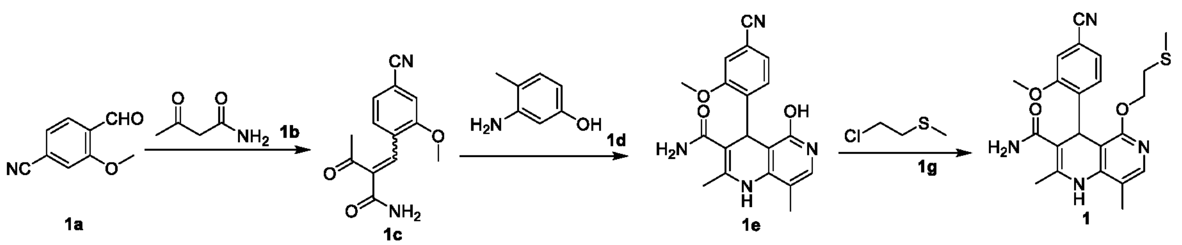


1a 1c 1e 1

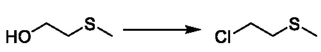

1f 1g

Step 1: Synthesis of compound 1c

Compound **1a** (3.0 g, 18.6 mmol) and compound 1b (2.82 mg, 27.9 mmol) were dissolved in dichloromethane (20 mL), and acetic acid (0.1 g) and morpholine (0.1 g) were added. The mixture was stirred at 40 °C until the reaction was complete. The mixture was cooled to room temperature and then filtered to give compound **1c** (3.5 g, 77.1% yield).

$^1$H NMR (400MHz, DMSO-d$^6$) $\delta$ = 7.86 (s, 1H), 7.76 (d, *J*=8.0 Hz, 1H), 7.62 (s, 1H), 7.59 (s, 2H), 7.48 (dd, *J*=1.1, 7.9 Hz, 1H), 3.93 (s, 3H), 2.36 (s, 3H).

Step 2: Synthesis of compound **1e**

Compound **1c** (1.0 g, 4.1 mmol) and compound **1d** (0.51 g, 4.1 mmol) were dissolved in isopropanol (10 mL), and the mixture was stirred at 95 °C until the reaction was complete. The solvent was removed under vacuum to give a crude product, and the crude product was purified by silica gel flash column chromatography (eluent: 0-10% methanol in dichloromethane) to give compound **1e** (0.9 g, 63.0% yield).

$^1$H-NMR (400MHz, DMSO-d$^6$) $\delta$ = 10.63 (br s, 1H), 7.54 (s, 1H), 7.41-7.35 (m, 2H), 7.28 (dd, *J*=1.3, 7.8 Hz, 1H), 7.12 (s, 1H), 6.93 (s, 1H), 6.88-6.57 (m, 2H), 3.80 (s, 3H), 2.11 (s, 3H), 2.01 (s, 3H).

Step 3: Synthesis of compound **1g**

Compound **If** (0.92 g, 10 mmol) was dissolved in chloroform (20 mL), and thionyl chloride (2 mL) was added. The mixture was stirred at 70 °C until the reaction was complete. The solvent was removed under vacuum to give a crude product, and the crude product was purified by silica gel flash column chromatography (eluent: 0-5% ethyl acetate in petroleum ether) to give compound **1g** (0.75 g, 68.2% yield).

Step 4: Synthesis of compound **1**

Compound **1e** (35 mg, 0.1 mmol), compound **1g** (16 mg, 1.5 mmol), and cesium carbonate (66 mg, 2 mmol) were dissolved in DMF (0.5 mL), and the mixture was stirred at 80 °C until the reaction was complete. The solvent was removed under vacuum to give a crude product, and the crude product was purified by reversed-phase preparative chromatography (eluent: 10-50% acetonitrile in water) to give compound **1** (4.6 mg, 10.8% yield).

$^1$H NMR (400MHz, $CD_3OD$) $\delta$ = 7.49 (s, 1H), 7.16 (s, 1H), 7.15-7.06 (m, 3H), 5.42 (s, 1H), 4.16-4.07 (m, 2H), 3.82 (s, 3H), 2.51 (dt, J=2.8, 6.7 Hz, 2H), 2.18 (s, 3H), 2.09 (s, 3H), 1.94 (s, 3H).

Example 2

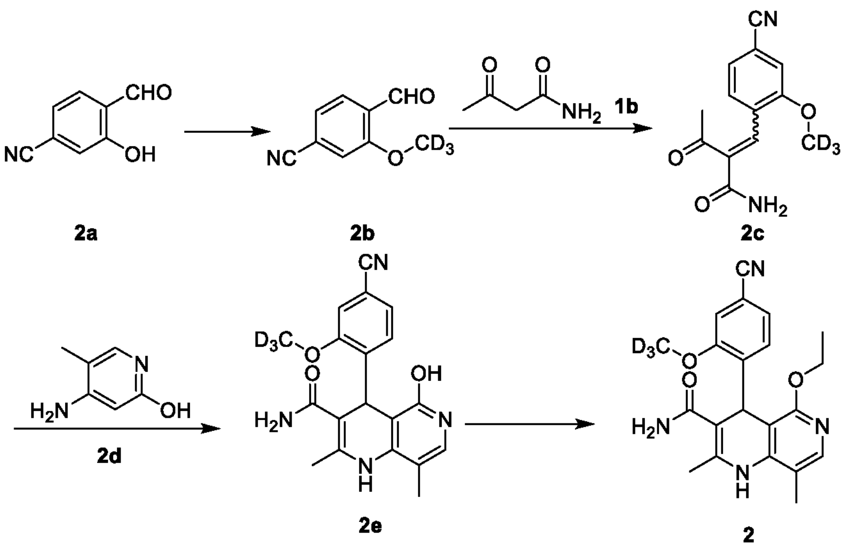


**2a** **2b** **2c** **2e** **2**

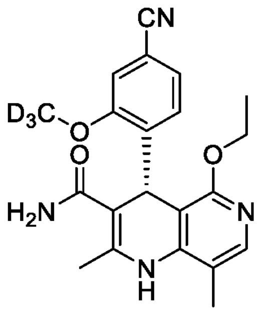 and 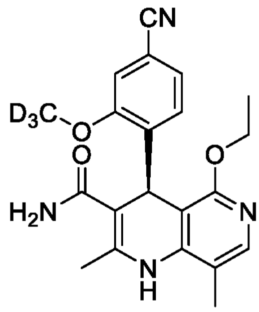 (compounds **2-1** and **2-2**)

Step 1: Synthesis of compound **2b**

Compound **2a** (1.0 g, 6.80 mmol) and potassium carbonate (1.0 g, 7.25 mmol) were mixed in dichloromethane (20 mL), and deuterated iodomethane (1.18 g, 8.16 mmol) was added. The mixture was stirred at room temperature until the reaction was complete. The mixture was cooled to room temperature and then diluted with water (20 mL). The mixture was extracted with ethyl acetate (20 mL × 3). The organic phases were separated, combined, washed with brine (20 mL), dried over anhydrous sodium sulfate, and then filtered. The filtrate was concentrated *in vacuo* to give a crude product, and the crude product was purified by flash column chromatography (eluent: 5-20% ethyl acetate in petroleum ether) to give compound **2b** (880 mg, 78.9% yield).

$^1$H-NMR (400 MHz, $CDCl_3$) δ ppm 10.50 (s, 1H), 7.92 (d, *J*=8.0 Hz, 1H), 7.34 (d, *J*=8.0 Hz, 1H), 7.27 (s, 1H).

Step 2: Synthesis of compound **2e**

Compound **2e** was prepared by using the method described in Example 1 and using compound **2b** as the starting material.

ES-MS m/z 354.2 $(M+H)^+$.

Step 3: Synthesis of compound **2**

Compound **2e** (260 mg, 0.736 mmol) was dissolved in NMP (3 mL), and sulfuric acid (36.11 mg, 0.368 mmol) and triethoxymethane (3 mL, 16.366 mmol) were added. The mixture was microwaved at 140 °C until the reaction was complete. The mixture was cooled to room temperature and then diluted with water (5 mL). The mixture was extracted with ethyl acetate (10 mL × 3). The organic phases were separated, combined, washed with brine (10 mL), dried over anhydrous sodium sulfate, and then filtered. The filtrate was concentrated *in vacuo* to give a crude product, and the crude product was purified by flash column chromatography (eluent: 0-60% ethyl acetate in petroleum ether) to give compound **2** (205 mg, 73.0% yield).

LCMS: m/z 382.2 $(M+H)^+$.

$^1$H NMR: (400 MHz, DMSO-$d^6$) δ ppm 7.69 (s, 1H), 7.55 (s, 1H), 7.37 (d, *J*= 1.6 Hz, 1H), 7.27 (dd, *J* = 1.6, 8.0 Hz, 1H), 7.15 (d, *J* = 8.0 Hz, 1H), 6.76-6.69 (m, 2H), 5.38 (s, 1H), 4.05-3.97 (m, 2H), 2.19 (s, 3H), 2.12 (s, 3H), 1.05 (t, *J*= 6.8 Hz, 3H).

The product was chirally resolved (column: ChiralPakAD (150 × 4.6 mm I.D., 5 μm); mobile phases: A: supercritical $CO_2$ fluid, B: ethanol (0.05% DEA)) to give compound **2-1** and **2-2.**

**Compound 2-1** (retention time: 1.109 min)

LCMS: m/z 382.2 $[M+H]^+$.

$^1$H NMR: (400 MHz, DMSO-$d^6$) δ ppm 7.68 (s, 1H), 7.55 (s, 1H), 7.36 (d, *J*= 1.6 Hz, 1H), 7.27 (dd, *J* = 1.6, 7.6 Hz, 1H), 7.14 (d, *J* = 7.6 Hz, 1H), 6.83-6.59 (m, 2H), 5.37 (s, 1H), 4.04-3.97 (m, 2H), 2.18 (s, 3H), 2.12 (s, 3H), 1.04 (t, *J*= 7.2 Hz, 3H).

**Compound 2-2** (retention time: 1.653 min)

LCMS: 382.2 $[M+H]^+$.

$^1$H NMR: (400 MHz, DMSO-$d^6$) δ ppm 7.68 (s, 1H), 7.55 (s, 1H), 7.36 (d, *J*= 1.6 Hz, 1H), 7.27 (dd, *J* = 1.6, 7.6 Hz, 1H), 7.14 (d, *J* = 7.6 Hz, 1H), 6.86-6.55 (m, 2H), 5.37 (s, 1H), 4.08-3.95 (m, 2H), 2.18 (s, 3H), 2.12 (s, 3H), 1.04 (t, *J*= 7.2 Hz, 3H).

Example 3

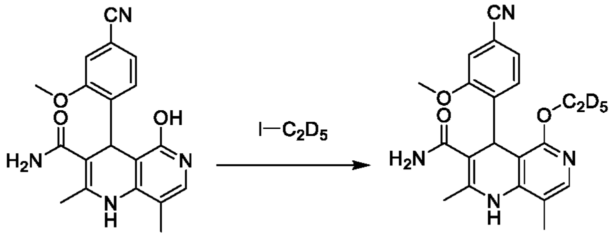


1e

3

Step 1: Synthesis of compound **3**

Compound **3** was prepared by using the method described in Example 1 and using compound **1e** as the starting material.

$^1$H-NMR (400MHz, DMSO-d6) δ = 7.69 (s, 1H), 7.56 (s, 1H), 7.37 (d, *J*=1.3 Hz, 1H), 7.28 (dd, J=1.3, 7.8 Hz, 1H), 7.15 (d, J=7.8 Hz, 1H), 6.89-6.62 (m, 2H), 5.38 (s, 1H), 3.83 (s, 3H), 2.19 (s, 3H), 2.12 (s, 3H).

Example 4

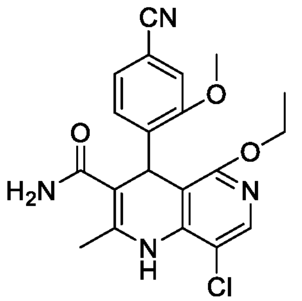

**4**

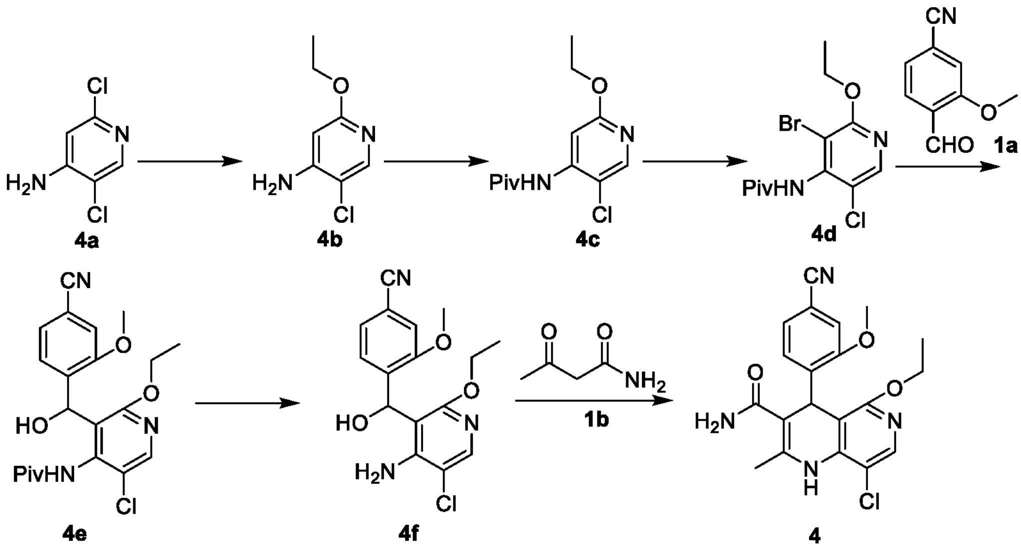

4a 4b 4c 4d 1a

4e 4f 1b 4

Step 1

Compound 4a (600 mg, 3.68 mmol) and potassium fluoride (427 mg, 7.36 mmol) were mixed in a 30% solution of sodium ethoxide in ethanol (3 mL), and the mixture was microwaved and stirred at 130 °C until the reaction was complete. After the mixture was cooled to room temperature, a saturated ammonium chloride solution (5 mL) was added, and the mixture was extracted with ethyl acetate (10 mL × 3). The combined organic phases were washed with water (10 mL) and brine (10 mL) and dried over anhydrous sodium sulfate, and the solvent was removed under vacuum to give a crude product. The crude product was purified by silica gel flash column chromatography (eluent: 0-30% ethyl acetate in petroleum ether) to give compound 4b (610 mg, 86.4% yield).

$^{1}$H NMR (400MHz, $CDCl_3$) δ = 7.88 (s, 1H), 6.02 (s, 1H), 4.47 (br s, 2H), 4.27 (q, J=7.1 Hz, 2H), 1.35 (t, J=7.1 Hz, 3H).

Step 2

Compound 4b (3.7 g, 21.4 mmol), triethylamine (6.0 mL, 43 mmol), and pivaloyl chloride (5.3 mL, 43 mmol) were mixed in dichloromethane (30 mL), and the solution was stirred at room temperature until the reaction was complete. Water (20 mL) was added, and the mixture was extracted with dichloromethane (30 mL × 3). The combined organic phases were washed with water (30 mL) and brine (30 mL) and dried over anhydrous sodium sulfate, and the solvent was removed under vacuum to give a crude product. The crude product was purified by silica gel flash column chromatography (eluent: 0-30% ethyl acetate in petroleum ether) to give compound 4c (6.0 g, 98.1% yield).

$^{1}$H NMR (400MHz, $CDCl_3$) δ = 8.10 (br s, 2H), 7.92 (br s, 1H), 4.33 (br d, *J=5.5* Hz, 2H), 1.38 (br d, *J*=5.4 Hz, 3H), 1.35 (s, 9H).

Step 3

Compound 4c (3.0 g, 11.7 mmol) was dissolved in DMF (30 mL) and acetic acid (0.2 mL), and NBS (2.5 g, 14.0 mmol) was added. The solution was stirred at 70 °C until the reaction was complete. After the mixture was cooled to room temperature, water (30 mL) was added, and the mixture was extracted with ethyl acetate (50 mL × 3). The combined organic phases were washed with water (50 mL × 2) and brine (50 mL) and dried over anhydrous sodium sulfate, and the solvent was removed under vacuum to give a crude product. The crude product was purified by silica gel flash column chromatography (eluent: 0-15% ethyl acetate in petroleum ether) to give compound 4d (3.1 g, 71.1% yield).

$^{1}$H NMR (400MHz, $CDCl_3$) δ = 8.08 (s, 1H), 7.25 (br s, 1H), 4.43 (q, J=7.0 Hz, 2H), 1.43 (t, *J*=7.0 Hz, 3H), 1.38 (s, 9H).

Step 4

Compound 4d (850 mg, 2.53 mmol) was dissolved in tetrahydrofuran (3 mL), and the solution was cooled to -78 °C. n-Butyllithium (1.0 M, 5.5 mL, 5.5 mmol) was added dropwise, and a solution of compound 1a (449 mg, 2.79 mmol) in tetrahydrofuran (2 mL) was added. The mixture was warmed to room temperature and stirred until the reaction was complete. Water (10 mL) was added, and the mixture was extracted with ethyl acetate (10 mL × 3). The combined organic phases were washed with water (10 mL) and brine (10 mL) and dried over anhydrous sodium sulfate, and the solvent was removed under vacuum to give a crude product. The crude product was purified by silica gel flash column chromatography (eluent: 0-20% ethyl acetate in petroleum ether) to give compound 4e (475 mg, 43.1% yield).

LCMS: m/z 418.2 $(M+H)^+$.

Step 5

Compound 4e (380 mg, 0.91 mmol) was dissolved in dioxane (3 mL), and concentrated hydrochloric acid (2 mL) and water (2 mL) were added. The solution was stirred at 85 °C until the reaction was complete. The mixture was cooled to room temperature, then made neutral with 2 N sodium hydroxide, and extracted with ethyl acetate (7 mL × 3). The combined organic phases were washed with water (10 mL) and brine (10 mL) and dried over anhydrous sodium sulfate, and the solvent was removed under vacuum to give a crude product. The crude product was purified by silica gel flash column chromatography (eluent: 0-30% ethyl acetate in petroleum ether) to give compound 4f (40 mg, 11.9% yield).

$^{1}$H NMR (400MHz, DMSO-$d^6$) δ = 7.75 (s, 1H), 7.57 (d, *J*=7.6 Hz, 1H), 7.40-7.37 (m, 2H), 6.30 (d, *J*=4.8 Hz, 1H), 6.20 (d, *J*=7.6 Hz, 1H), 6.07 (s, 2H), 4.18-4.08 (m, 2H), 3.77 (s, 1H), 1.17 (t, *J*= 7.0 Hz, 3H).

Step 6

Compound 4f (15 mg, 0.045 mmol) and compound 1b (14 mg, 0.135 mmol) were dissolved in toluene (1 mL), and diphenylphosphoric acid (2.2 mg, 0.01 mmol) was added. The solution was stirred at 110 °C until the reaction was complete. After the mixture was cooled to room temperature, water (1 mL) was added, and the mixture was extracted with ethyl acetate (2 mL $\times$ 3). The combined organic phases were washed with water (2 mL) and brine (2 mL) and dried over anhydrous sodium sulfate, and the solvent was removed under vacuum to give a crude product. The crude product was purified by silica gel flash column chromatography (eluent: 0-10% methanol in dichloromethane) to give compound 4 (2 mg, 11.1% yield).

$^1$H NMR (400MHz, $CD_3OD$) $\delta$ = 7.79 (s, 1H), 7.26-7.19 (m, 3H), 5.51 (s, 1H), 4.15-4.03 (m, 2H), 3.88 (s, 3H), 2.23 (s, 3H), 1.13 (t, J=7.2 Hz, 3H).

Example 5

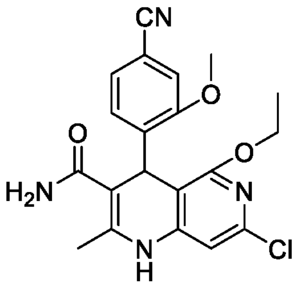

**5**

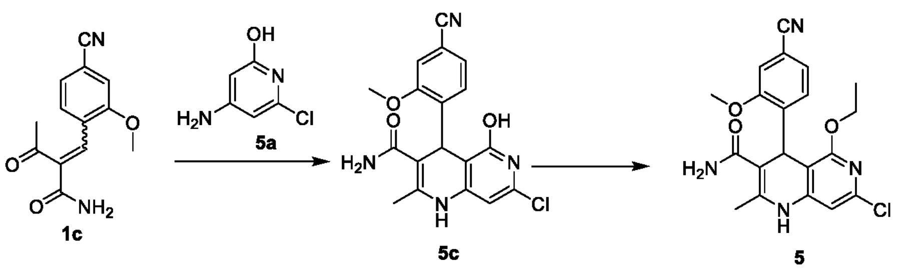

1c 5a 5c 5

Compound 5 was prepared by using the method described in Example 2 and using compounds 1c and 5a as the starting materials.

$^1$H NMR (400 MHz, DMSO-$d^6$) $\delta$ = 8.97-8.87 (m, 1H), 7.38 (s, 1H), 7.29 (dd, *J*= 1.3, 7.6 Hz, 1H), 7.19 (d, *J* = 7.6 Hz, 1H), 6.81-6.58 (m, 2H), 6.44 (s, 1H), 5.34 (s, 1H), 4.08-3.98 (m, 2H), 3.82 (s, 3H), 2.10 (s, 3H), 1.06 (t, *J*=7.6 Hz, 3H).

Example 6

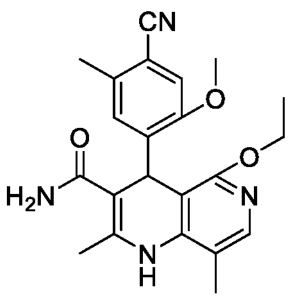

**6**

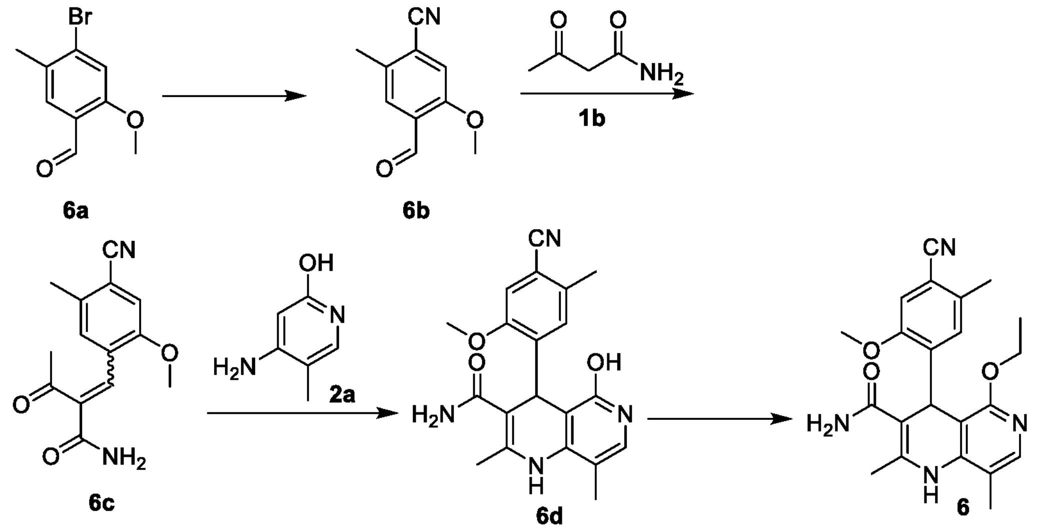


6a 6b 6c 6d 6

Step 1

Compound 6a (5.6 g, 24.4 mmol), potassium hexacyanoferrate(III) (12.1 g, 36.7 mmol), palladium acetate (1.1 g, 4.9 mmol), and sodium carbonate (5.18 g, 48.9 mmol) were mixed in DMF (70 mL), and the mixture was microwaved and stirred at 120 °C in a nitrogen atmosphere until the reaction was complete. After the mixture was cooled to room temperature, 80 mL of water was added, and the mixture was extracted with ethyl acetate (80 mL × 3). The combined organic phases were washed with brine (80 mL), dried over anhydrous sodium sulfate, and filtered. The solvent was removed under vacuum to give a crude product, and the crude product was purified by silica gel flash column chromatography (eluent: 0-40% ethyl acetate in petroleum ether) to give compound 6b (300 mg, 7.0% yield).

$^1$H NMR (400MHz, $CDCl_3$) $\delta$ = 10.47 (s, 1H), 7.75 (s, 1H), 7.21 (s, 1H), 3.96 (s, 3H), 2.52 (s, 3H).

Step 2

Compound 6 was prepared by using the method described in Example 2 and using compounds 6b and 1b as the starting materials.

$^1$H NMR (400MHz, DMSO-d$^6$) $\delta$ = 7.67 (s, 1H), 7.55 (s, 1H), 7.29 (s, 1H), 7.02 (s, 1H), 6.73 (br s, 2H), 5.34 (s, 1H), 4.01 (dd, J=4.8, 7.2 Hz, 2H), 3.78 (s, 3H), 2.28 (s, 3H), 2.20 (s, 3H), 2.12 (s, 3H), 1.06 (t, J=7.2 Hz, 3H).

Test Example 1: Assay for *In-Vitro* Antagonistic Activity Against Mineralocorticoid Receptors

1) Reagent preparation

All the compounds to be tested were serially diluted 3-fold from 10 mM with DMSO (Sigma, D8418), and 10 concentrations were obtained for each of the compounds.

The reference compound eplerenone was serially diluted 3-fold with DMSO to form 10 concentrations.

A 1000× positive control (10 mM eplerenone) and a 1000× negative control (100% DMSO) were prepared.

2) Experimental procedure

2.1. All cells were cultured as recommended by ATCC. HEK293T cells were passaged and plated when in the exponential growth phase.

2.2. The old culture medium in the cell culture flask was discarded, and the cells were washed with PBS.

2.3. A proper amount of TrypLE solution was added to the culture flask to digest the cells to separate them, and the digestion was stopped with a serum-containing complete culture medium.

2.4. The cell suspension was centrifuged to make the cells settle. The cells were washed with PBS 2 times to remove phenol red and were then suspended with culture medium to a proper concentration (only cells with a survival rate of greater than 90% were used in the subsequent experiments).

2.5. 6 × 106 HEK293T cells were spread on a 100 mm Petri dish and incubated in a 37 °C, 5% $CO_2$ incubator for 16 h.

2.6. The cells were transfected with plasmids and then incubated in a 37 °C, 5% $CO_2$ incubator for another 5-6 h.

2.7. 25 nL of compound dilution was transferred to a 384-well assay plate using Echo655.

2.8. HEK293T cells were plated at a concentration of 17,000 cells/well in a 384-well plate, and meanwhile 1 nM aldosterone was added to each well.

2.9. The cells were incubated in a 37 °C, 5% $CO_2$ incubator for 18-20 h.

2.10. 25 μL of britelite + luciferase assay reagent was added to each well of the 384-well assay plate, and luminescence values were recorded on an Envision 2105 plate reader.

2.11. The IC50 values of the compounds were obtained using the software Graphad 8.0 and a non-linear fitting equation.

Table 1

| No. | IC50/ nM |
|---|---|
| Compound 1 | 16 |
| Compound 2 | 7 |
| Compound 3 | 17 |
| Compound 4 | 11 |
| Compound 5 | 5 |
| Compound 6 | 10 |
| Finerenone | 13 |

Test Example 2: Human Liver Microsome Metabolism Study

222.5 μL of human liver microsomes (protein concentration: 1 mg/mL) and 25 μL of NADPH (10 nM) were added to an incubation plate and pre-heated for 10 min. 2.5 μL of control compound and test compound (100 μM) were added.

30-μL aliquots were taken from the reaction solution at 0.5, 5, 10, 15, 20, and 30 min. The reaction was stopped by adding 5 volumes of cold acetonitrile containing IS (100 nM alprazolam, 200 nM caffeine, and 100 nM tolbutamide). The reaction solution was centrifuged, and 100 μL of the supernatant was collected and mixed with 100 μL of ultrapure $H_2O$ before analysis by LC-MS/MS.

The slope value k was determined by linear regression of the natural logarithm of a curve of the parent drug remaining percentage versus incubation time.

The *in-vitro* half-life *(in-vitro* $T_{1/2}$) was determined by the slope value:

$$\mathrm{T1/2} = -(0.693/k)$$

Using the following equation (the average of repeated measurements), the *in-vitro* T1/2 (min) was converted to *in-vitro* intrinsic clearance *(in-vitro* $CL_{int}$, in μL/min/mg protein):

$$\mathrm{CL_{int}} = \left(\frac{0.693}{T1/2}\right) * \left(\frac{\text{incubation amount (μL)}}{\text{protein amount (mg)}}\right)$$

Table 2

| Time / No. | 0.5 min | 5 min | 10 min | 15 min | 20 min | 30 min |
|---|---|---|---|---|---|---|
| | Remaining amount% | | | | | |
| Compound 2-2 | 100.00 | 65.90 | 43.56 | 31.75 | 21.72 | 15.34 |
| Finerenone | 100.00 | 67.31 | 42.44 | 24.78 | 16.62 | 7.44 |

Table 3

| Parameter / No. | $T_{1/2}$ (min) | CLint (μL/min/mg) |
|---|---|---|
| Compound 2-2 | 10.79 | 64.20 |
| Finerenone | 7.40 | 93.66 |

Conclusion: The compounds of the disclosure, e.g., compound 2-2, have better metabolic stability than finerenone.

Test Example 3: Efficacy Test with db/db Mouse Model of Diabetic Nephropathy Proper amounts of compound **2-2** and finerenone were taken and separately mixed with a 0.5% hydroxyethyl cellulose solution (Tylose MH300) to form suspensions.

1) Grouping and administration

30 db/db mice (idiopathic type II diabetes model mice) were selected and divided into a model control group, a positive control group, and an administration group, and 10 db/m mice were selected as a control group.

The mice were dosed by oral gavage (i.g) once daily over 4 consecutive weeks. The dosing regimen is shown in detail in Table 4.

Table 4. Dosing regimen

| Group | Route of administration | Dose (mg/kg) | Concentration (mg/mL) | Volume (mL/kg) | Number of animals |
|---|---|---|---|---|---|
| Control group | i.g. | - | - | 10 | 10 |
| Model control group | | - | - | 10 | 10 |
| Positive control group | | 15 | 1.5 | 10 | 10 |
| Administration group | | 15 | 1.5 | 10 | 10 |

2) Evaluation measures

Urine albumin was used as a main measure. The data obtained were analyzed statistically using EXCEL and IBM SPSS Statistics 22.0.

All metrology data are expressed as Mean ± SEM s. The parameters before and after dosing were plotted for the different groups of animals using the software Graph Pad Prism 8. Data were analyzed using the statistical software SPSS 22.0. A Levene's test of homogeneity of variance was performed on the parameters. When the variance was homogenous ($P \geq 0.05$), the Dunnett's & LSD method from the one-way analysis of variance (ANOVA) was used to compare the differences between the groups. When the variance was not homogenous ($P < 0.05$), the Mann-Whitney U test (the M-W method) from the Kruskal-Wallis H test (the K-W method) was used to compare the differences between the groups. For animal survival, the log-rank test from the product-limit method (the K-M method) was used to compare the differences between the groups.

3) Experimental results

Table 5

| Group | Urine weight (mL) | | mALB (μg/24h) | | Urine creatinine (μmol/24 h) | | UACR (μg/mg) | |
|---|---|---|---|---|---|---|---|---|
| | 0 weeks | 4 weeks | 0 weeks | 4 weeks | 0 weeks | 4 weeks | 0 weeks | 4 weeks |
| Control group | 1.05± 0.15 | 2.42± 0.24 | 100.26± 15.08 | 224.15± 23.32 | 0.40± 0.05 | 0.99± 0.03 | 1117.42± 117.99 | 1264.37± 231.49 |
| Model control group | 2.74± 0.14 | 15.15± 1.31 | 226.53± 14.04 | 1,325.39± 125.62 | 0.36± 0.03 | 2.10± 0.23 | 5554.89± 238.38 | 5821.39± 485.53 |
| Positive control group | 2.86± 0.17 | 11.96± 2.89 | 226.68± 13.90 | 1,013.76± 245.40 | 0.37± 0.02 | 2.11± 0.44 | 5538.19± 342.61 | 4294.14± 464.95 |
| Administration group | 2.72± 0.14 | 10.41± 2.29 | 224.72± 13.19 | 870.27± 189.40 | 0.40± 0.01 | 2.23± 0.39 | 5039.91± 334.27 | 3301.13± 330.31** |

Note: (compared to the model control group, * $P < 0.05$, and ** $P < 0.01$)

Conclusion: After 4 weeks of administration, both the urine amount and mALB in the finerenone group and the administration group (compound 2) tended to be lower than those in the model control group. Meanwhile, the urine albumin-to-creatinine ratio (UACR) of the mice in the administration group is statistically significantly smaller than that of the mice in the model control group ($P < 0.01$); the urine albumin-to-creatinine ratio (UACR) of the mice in the finerenone group decreased somewhat but is not significantly different from that of the mice in the model group, as shown in FIG. 1 (compared to the model control group, ** $P < 0.01$).

## Claims

1. A compound represented by formula I or a pharmaceutically acceptable salt or isotopically substituted form thereof,

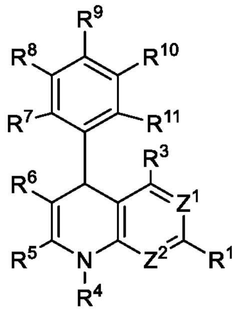

I

,

wherein $R^1$ is selected from the group consisting of hydrogen, halogen, $C_{1-6}$ alkyl, $C_{1-6}$ alkoxy, 3- to 6-membered cycloalkyl, and 3- to 6-membered heterocycloalkyl; the alkyl, alkoxy, cycloalkyl, or heterocycloalkyl is optionally substituted with one or more $R^{1A}$, and each $R^{1A}$ is independently selected from the group consisting of halogen, hydroxy, cyano, and amino;

$Z^1$ and $Z^2$ are each independently selected from the group consisting of N and C-$R^2$, and $Z^1$ and $Z^2$ are not simultaneously C-$R^2$;

$R^2$ is selected from the group consisting of hydrogen, halogen, $C_{1-6}$ alkyl, $C_{1-6}$ alkoxy, 3-to 6-membered cycloalkyl, and 3- to 6-membered heterocycloalkyl; the alkyl, alkoxy, cycloalkyl, or heterocycloalkyl is optionally substituted with one or more $R^{2A}$, and each $R^{2A}$ is independently selected from the group consisting of halogen, hydroxy, cyano, and amino;

when $Z^1$ is N, $R^3$ is selected from the group consisting of the following functional groups:

1) -S-$C_{1-6}$ alkyl, -S-3- to 6-membered cycloalkyl, and -S-3- to 6-membered heterocycloalkyl, wherein the alkyl, cycloalkyl, or heterocycloalkyl is optionally substituted with halogen, $C_{1-6}$ alkoxy, 3- to 6-membered cycloalkyl, or 3- to 6-heterocycloalkyl;
2) -O-$C_{1-6}$ alkyl, wherein the alkyl is substituted with one or more $R^{8A}$, and each $R^{8A}$ is independently selected from the group consisting of $C_{1-6}$ alkoxy, 3- to 6-membered cycloalkyl, 3- to 6-membered heterocycloalkyl, 3- to 6-membered cycloalkoxy, 3- to 6-membered heterocycloalkoxy, $C_{1-6}$ alkylthio, 3- to 6-membered cycloalkylthio, and 3-to 6-membered heterocycloalkylthio; the alkoxy, cycloalkyl, heterocycloalkyl, cycloalkoxy, heterocycloxy, alkylthio, cycloalkylthio, or heterocycloalkylthio is optionally substituted with halogen, hydroxy, cyano, or amino; and
3) -O-$C_{1-6}$ alkyl, wherein the alkyl is substituted with fluorine at least three times; when $Z^1$ is C-$R^2$, $R^3$ is selected from the group consisting of halogen, $C_{1-6}$ alkyl, $C_{1-6}$ alkoxy, 3- to 6-membered cycloalkyl, 3- to 6-membered cycloalkoxy, 3- to 6-membered heterocycloalkyl, and 3- to 6-membered heterocycloalkoxy, wherein the alkyl, alkoxy, cycloalkyl, cycloalkoxy, heterocycloalkyl, or heterocycloalkoxy is optionally substituted with one or more $R^{3A}$, and each $R^{3A}$ is independently selected from the group consisting of halogen, hydroxy, cyano, amino, $C_{1-6}$ alkyl, $C_{1-6}$ alkoxy, 3- to 6-membered cycloalkyl, 3- to 6-membered cycloalkoxy, 3- to 6-membered heterocycloalkyl, and 3-to 6-membered heterocycloalkoxy, wherein the alkyl, alkoxy, cycloalkyl, cycloalkoxy, heterocycloalkyl, or heterocycloalkoxy is optionally substituted with one or more groups selected from the group consisting of halogen, hydroxy, cyano, and amino;

$R^4$ is selected from the group consisting of hydrogen, $C_{1-6}$ alkyl, 3- to 6-membered cycloalkyl, and 3- to 6-membered heterocycloalkyl; the alkyl, alkoxy, cycloalkyl, or heterocycloalkyl is optionally substituted with one or more $R^{4A}$, and each $R^{4A}$ is independently selected from the group consisting of halogen, hydroxy, cyano, and amino;
$R^5$ is selected from the group consisting of hydrogen, halogen, $C_{1-6}$ alkyl, $C_{1-6}$ alkoxy, 3-to 6-membered cycloalkyl, and 3- to 6-membered heterocycloalkyl; the alkyl, alkoxy, cycloalkyl, or heterocycloalkyl is optionally substituted with one or more $R^{5A}$, and each $R^{5A}$ is independently selected from the group consisting of halogen, hydroxy, cyano, and amino;
$R^6$ is selected from the group consisting of hydrogen, halogen, $C_{1-6}$ alkyl, NR'(R"), COOR', and CONR'(R"); the alkyl is optionally substituted with one or more $R^{6A}$, and each $R^{6A}$ is independently selected from the group consisting of halogen, hydroxy, cyano, and amino;
R' or R" is independently selected from the group consisting of hydrogen, $C_{1-6}$ alkyl, 3-to 6-membered cycloalkyl, and 3- to 6-membered heterocycloalkyl; the alkyl, alkoxy, cycloalkyl, or heterocyclyl is optionally substituted with one or more groups selected from the group consisting of halogen, hydroxy, cyano, and amino;
$R^7$, $R^8$, $R^9$, $R^{10}$, and $R^{11}$ are independently selected from the group consisting of hydrogen, halogen, hydroxy, cyano, nitro, amino, $C_{1-6}$ alkyl, $C_{1-6}$ alkoxy, 3- to 6-membered cycloalkyl, and 3- to 6-membered heterocycloalkyl; the alkyl, alkoxy, cycloalkyl, or heterocycloalkyl is optionally substituted with one or more $R^{7A}$, and each $R^{7A}$ is independently selected from the group consisting of halogen, hydroxy, oxo, nitro, cyano, and amino.

2. The compound or the pharmaceutically acceptable salt or isotopically substituted form thereof according to claim 1, wherein $R^1$ is selected from the group consisting of hydrogen, $C_{1-6}$ alkyl, and 3- to 6-membered cycloalkyl, preferably from the group consisting of hydrogen and $C_{1-6}$ alkyl, for example, from the group consisting of hydrogen, methyl, difluoromethyl, trifluoromethyl, ethyl, and propyl; further, the alkyl or cycloalkyl is optionally substituted with 1-3 $R^{1A}$, and $R^{1A}$ is as defined in claim 1.

3. The compound or the pharmaceutically acceptable salt or isotopically substituted form thereof according to claim 1 or 2, wherein $R^6$ is selected from the group consisting of COOR' and CONR'(R"), preferably from CONR'(R"), and R' or R" is as defined in claim 1.

4. The compound or the pharmaceutically acceptable salt or isotopically substituted form thereof according to any of claims 1-3, wherein $R^9$ is selected from the group consisting of cyano and nitro.

5. The compound or the pharmaceutically acceptable salt or isotopically substituted form thereof according to any of claims 1-4, wherein the compound represented by formula I is

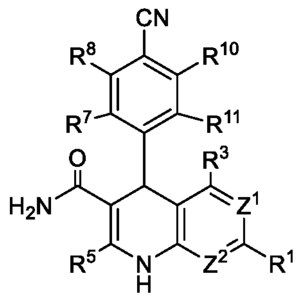

II,

wherein $R^1$, $R^3$, $R^5$, $R^7$, $R^8$, $R^{10}$, $R^{11}$, $Z^1$, and $Z^2$ are as defined in claim 1.

**6.** The compound or the pharmaceutically acceptable salt or isotopically substituted form thereof according to any of claims 1-5, wherein $R^5$ is selected from the group consisting of hydrogen, halogen, $C_{1-6}$ alkyl, and $C_{1-6}$ alkoxy, preferably from the group consisting of hydrogen and $C_{1-6}$ alkyl, for example, from the group consisting of hydrogen, methyl, ethyl, and propyl; further, the alkyl or alkoxy is optionally substituted with 1-3 $R^{5A}$, and $R^{5A}$ is as defined in claim 1.

**7.** The compound or the pharmaceutically acceptable salt or isotopically substituted form thereof according to any of claims 1-6, wherein $R^7$ is selected from the group consisting of hydrogen, halogen, $C_{1-6}$ alkyl, and $C_{1-6}$ alkoxy, preferably from the group consisting of hydrogen, $C_{1-6}$ alkyl, and $C_{1-6}$ alkoxy, for example, from the group consisting of hydrogen, methyl, difluoromethyl, trifluoromethyl, ethyl, propyl, methoxy, ethoxy, difluoromethoxy, and trifluoromethoxy; further, the alkyl or alkoxy is optionally substituted with 1-3 $R^{7A}$, and $R^{7A}$ is as defined in claim 1.

**8.** The compound or the pharmaceutically acceptable salt or isotopically substituted form thereof according to any of claims 1-7, wherein the compound represented by formula I is

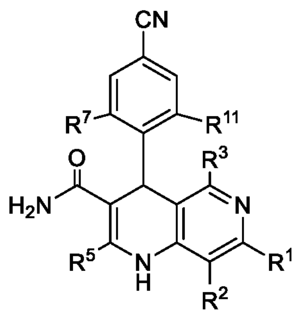

IIIa,

wherein $R^1$, $R^2$, $R^3$, $R^5$, $R^7$, and $R^{11}$ are as defined in claim 1.

**9.** The compound or the pharmaceutically acceptable salt or isotopically substituted form thereof according to any of claims 1-8, wherein $R^2$ is selected from the group consisting of hydrogen, halogen, $C_{1-6}$ alkyl, and $C_{1-6}$ alkoxy, preferably from the group consisting of hydrogen and $C_{1-6}$ alkyl, for example, from the group consisting of hydrogen, methyl, difluoromethyl, trifluoromethyl, ethyl, and propyl; further, the alkyl or alkoxy is optionally substituted with 1-3 $R^{2A}$, and $R^{2A}$ is as defined in claim 1.

**10.** The compound or the pharmaceutically acceptable salt or isotopically substituted form thereof according to claim 1, wherein $R^1$ is selected from the group consisting of hydrogen, fluorine, chlorine, difluoromethyl, and trifluoromethyl.

**11.** The compound or the pharmaceutically acceptable salt or isotopically substituted form thereof according to claim 1, wherein $R^3$ is selected from the group consisting of the following functional groups:

1) -S-$C_{1-6}$ alkyl, wherein the alkyl is optionally substituted with halogen or $C_{1-6}$ alkoxy;
2) -O-$C_{1-6}$ alkyl, wherein the alkyl is substituted with one or more $R^{8A}$, and each $R^{8A}$ is independently selected from the group consisting of methylthio, difluoromethylthio, trifluoromethylthio, methoxy, difluoromethoxy, and trifluoromethylthio; and

3) -O-$C_{2-6}$ perfluoroalkyl.

**12.** The compound or the pharmaceutically acceptable salt or isotopically substituted form thereof according to claim 1, wherein the compound represented by formula I is selected from the group consisting of:

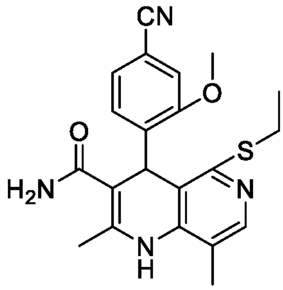
,
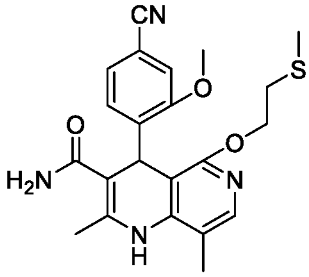
,
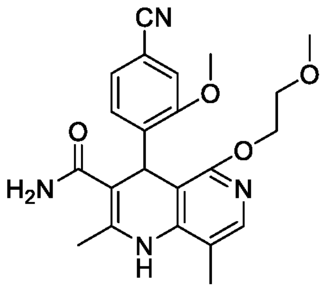
,
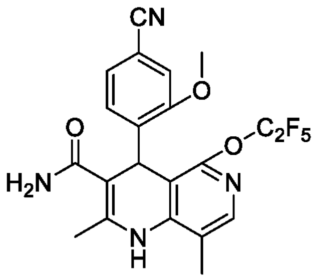
,
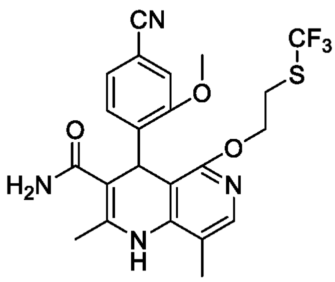
and
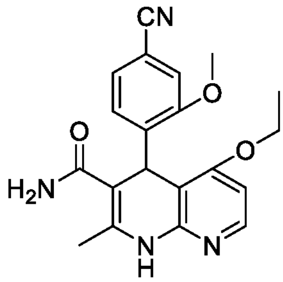
.

**13.** A compound or a pharmaceutically acceptable salt thereof, being selected from the group consisting of:

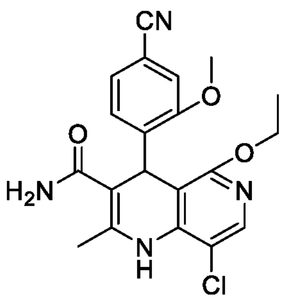
,
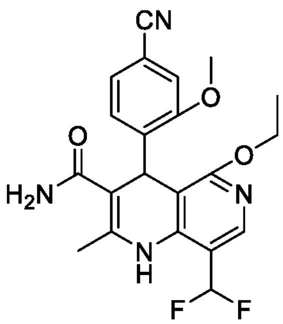
,
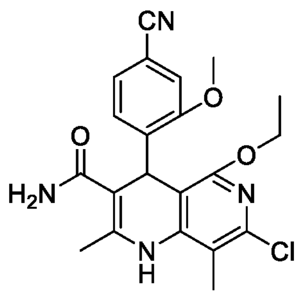
,
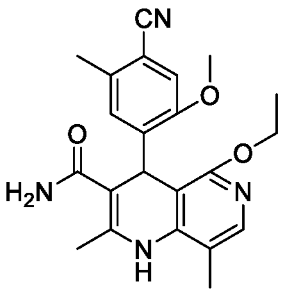
,
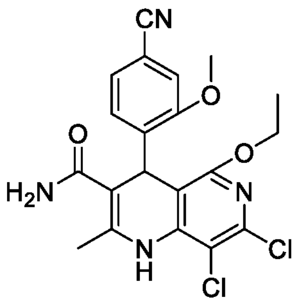
,
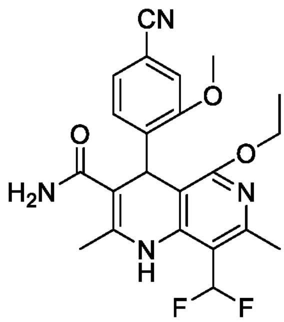
,

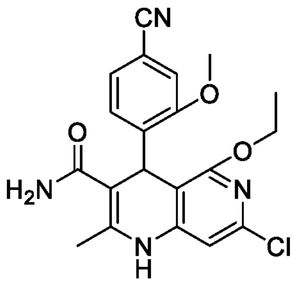,
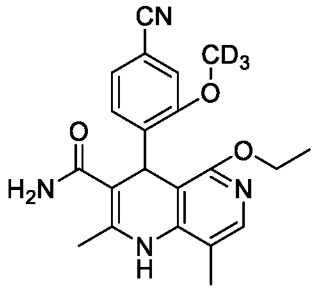,
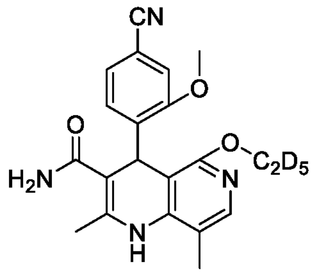,
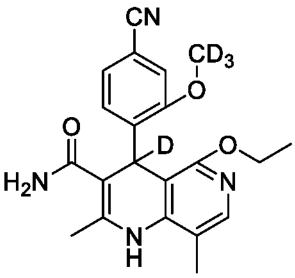,
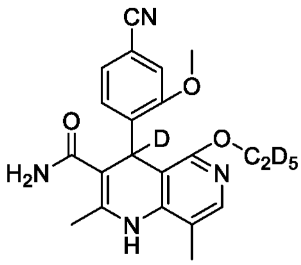,
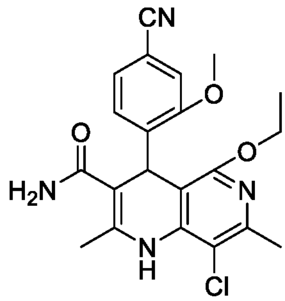,
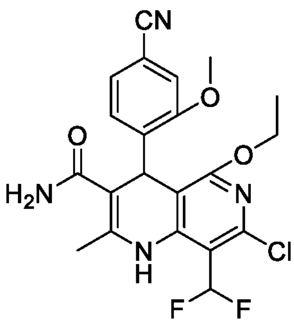
and
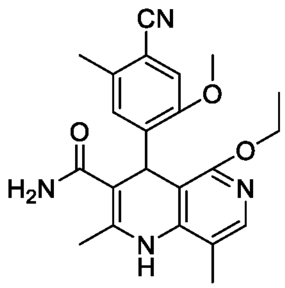.

**14.** The compound or the pharmaceutically acceptable salt thereof according to claim 13, having deuterium with an abundance that is at least 1000 times greater than the natural abundance of deuterium, preferably deuterium with an abundance that is at least 2000 times greater than the natural abundance of deuterium, and more preferably deuterium with an abundance that is at least 3000 times greater than the natural abundance of deuterium.

**15.** A pharmaceutical composition, comprising a therapeutically effective amount of at least one of the compound or the pharmaceutically acceptable salt or isotopically substituted form thereof according to any of claims 1-12, or the compound or the pharmaceutically acceptable salt thereof according to claim 13 or 14, and a pharmaceutically acceptable excipient.

**16.** Use of the compound or the pharmaceutically acceptable salt or isotopically substituted form thereof according to any of claims 1-12, or the compound or the pharmaceutically acceptable salt thereof according to claim 13 or 14, or the pharmaceutical composition according to claim 15 in the preparation of a medicament for preventing and/or treating a mineralocorticoid-related disease or disorder.

**17.** Use of the compound or the pharmaceutically acceptable salt or isotopically substituted form thereof according to any of claim 12, or the compound or the pharmaceutically acceptable salt thereof according to claim 13 or 14, or the pharmaceutical composition according to claim 15 in the preparation of a medicament for preventing and/or treating the disorders hyperaldosteronism, hypertension, and heart failure.

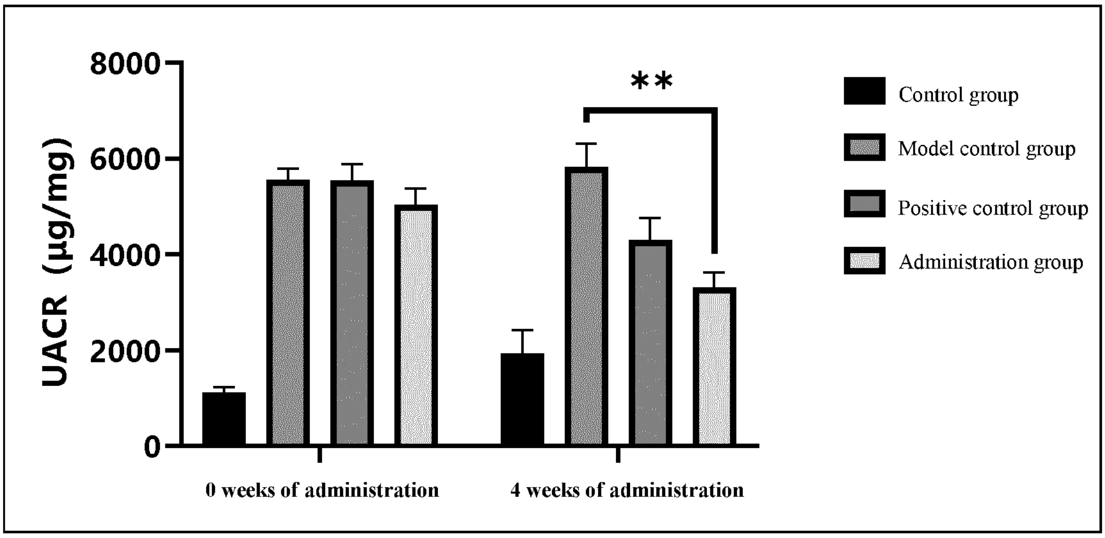
8000
6000
4000
2000
0
UACR (μg/mg)
**
Control group
Model control group
Positive control group
Administration group
0 weeks of administration
4 weeks of administration

FIG. 1

**INTERNATIONAL SEARCH REPORT**

International application No. **PCT/CN2022/119209**

**A. CLASSIFICATION OF SUBJECT MATTER**

C07D 471/04(2006.01)i; A61K 31/4375(2006.01)i; A61P 9/00(2006.01)i; A61P 9/12(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, EPODOC, WPI, STN-REGISTRY, STN-CAPLUS, STN-MARPAT: 上海拓界, 盐皮质激素受体, 醛甾酮, 萘啶, mineralocorticoid receptor, MR, aldosterone, +naphthyridine, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 110511219 A (GUANGDONG HEC PHARMACEUTICAL CO., LTD. et al.) 29 November 2019 (2019-11-29)<br>claims 1 and 11-15, and description, embodiments 1-3, 5-6, and 9-10 | 1-10, 15-17 |
| X | CN 101641352 A (BAYER HEALTHCARE AG) 03 February 2010 (2010-02-03)<br>description, pages 3-4 and 8 | 1-17 |
| Y | CN 101641352 A (BAYER HEALTHCARE AG) 03 February 2010 (2010-02-03)<br>description, pages 3-4 and 8 | 1-12, 15-17 |
| Y | US 2010305052 A1 (BAYER SCHERING PHARMA AKTIENGESELLSCHAFT) 02 December 2010 (2010-12-02)<br>description, paragraphs [0011]-[0039] and [0404] | 1-12, 15-17 |
| X | CN 113214248 A (HUNAN NUCIEN PHARMACEUTICAL CO., LTD. et al.) 06 August 2021 (2021-08-06)<br>description, paragraphs 7-15 | 13-17 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 November 2022** | **30 November 2022** |

Name and mailing address of the ISA/CN

**China National Intellectual Property Administration (ISA/CN)**
**No. 6, Xitucheng Road, Jimenqiao, Haidian District, Beijing 100088, China**

Facsimile No. **(86-10)62019451**

Authorized officer

Telephone No.

**INTERNATIONAL SEARCH REPORT**
**Information on patent family members**

International application No.
**PCT/CN2022/119209**

| Patent document cited in search report | Publication date (day/month/year) | Patent family member(s) | Publication date (day/month/year) |
|---|---|---|---|
| CN 110511219 A | 29 November 2019 | CA 3100271 A1 | 28 November 2019 |
| | | WO 2019223629 A1 | 28 November 2019 |
| | | AU 2019272300 A1 | 03 December 2020 |
| | | JP 2021524468 A | 13 September 2021 |
| | | US 2021206760 A1 | 08 July 2021 |
| | | EP 3798219 A1 | 31 March 2021 |
| CN 101641352 A | 03 February 2010 | PT 2132206 E | 04 August 2015 |
| | | SA 2982 B1 | 03 November 2012 |
| | | TN 2009000318 A1 | 31 December 2010 |
| | | HN 2009001597 A | 16 January 2012 |
| | | BR 122020008544 B1 | 17 February 2021 |
| | | HU E026441 T2 | 28 June 2016 |
| | | BR PI0808098 A2 | 17 June 2014 |
| | | IN 4992DELNP2009 A | 12 March 2010 |
| | | PE 20090724 A1 | 17 July 2009 |
| | | MY 150748 A | 28 February 2014 |
| | | SI 2132206 T1 | 31 August 2015 |
| | | CA 2679232 A1 | 04 September 2008 |
| | | JP 2014012678 A | 23 January 2014 |
| | | JO 3018 B1 | 05 September 2016 |
| | | JP 2010519232 A | 03 June 2010 |
| | | KR 20090129992 A | 17 December 2009 |
| | | GT 200900230 A | 29 November 2011 |
| | | ES 2540803 T3 | 13 July 2015 |
| | | AU 2008221071 A1 | 04 September 2008 |
| | | UA 102065 C2 | 10 June 2013 |
| | | ZA 200905730 B | 27 October 2010 |
| | | UY 30931 A1 | 30 September 2008 |
| | | CU 20090148 A7 | 26 April 2011 |
| | | UY 38953 A | 31 August 2021 |
| | | CR 10976 A | 13 January 2010 |
| | | US 2010136142 A1 | 03 June 2010 |
| | | HK 1140194 A1 | 08 October 2010 |
| | | EP 2132206 A2 | 16 December 2009 |
| | | LT PA2022512 I1 | 11 July 2022 |
| | | PA 8770101 A1 | 15 May 2009 |
| | | MY 176873 A | 25 August 2020 |
| | | RU 2009135659 A | 10 April 2011 |
| | | NZ 579230 A | 27 April 2012 |
| | | HU S2200015 I1 | 28 June 2022 |
| | | IL 200060 D0 | 15 April 2010 |
| | | MX 2009008701 A | 27 August 2009 |
| | | MA 31245 B1 | 01 March 2010 |
| | | DE 102007009494 A1 | 28 August 2008 |
| | | DK 2132206 T3 | 06 July 2015 |
| | | HR P20150702 T1 | 14 August 2015 |
| | | PL 2132206 T3 | 31 August 2015 |
| | | CO 6220951 A2 | 19 November 2010 |
| | | DO P2009000205 A | 28 February 2010 |
| | | WO 2008104306 A2 | 04 September 2008 |

Form PCT/ISA/210 (patent family annex) (January 2015)

**INTERNATIONAL SEARCH REPORT**
**Information on patent family members**

International application No.
**PCT/CN2022/119209**

| Patent document cited in search report | Publication date (day/month/year) | Patent family member(s) | Publication date (day/month/year) |
|---|---|---|---|
| | | TW 200843755 A | 16 November 2008 |
| | | EC SP099581 A | 29 September 2009 |
| | | UY 38952 A | 31 August 2021 |
| | | US 2014100243 A1 | 10 April 2014 |
| | | CL 2008000502 A1 | 13 June 2008 |
| US 2010305052 A1 | 02 December 2010 | CA 2654315 A1 | 13 December 2007 |
| | | AT 523510 T | 15 September 2011 |
| | | JP 2009539787 A | 19 November 2009 |
| | | DE 102006026585 A1 | 13 December 2007 |
| | | EP 2029591 A1 | 04 March 2009 |
| | | ES 2372626 T3 | 24 January 2012 |
| | | WO 2007140934 A1 | 13 December 2007 |
| CN 113214248 A | 06 August 2021 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008104306 A
- WO 2019223629 A
- WO 2007140934 A
- WO 2007140894 A
- CN 202110397352